(19) Europäisches Patentamt
European Patent Office
Office européen des brevets

(11) EP 3 407 872 B1

(12) **EUROPÄISCHE PATENTSCHRIFT**

(45) Veröffentlichungstag und Bekanntmachung des Hinweises auf die Patenterteilung:
**11.03.2020 Patentblatt 2020/11**

(21) Anmeldenummer: **17705303.0**

(22) Anmeldetag: **27.01.2017**

(51) Int Cl.:
***A61K 9/14*** *(2006.01)*

(86) Internationale Anmeldenummer:
**PCT/EP2017/000097**

(87) Internationale Veröffentlichungsnummer:
**WO 2017/129365 (03.08.2017 Gazette 2017/31)**

(54) **FUNKTIONALISIERTE METALLISCHE NANOPARTIKEL**

FUNCTIONALIZED METAL NANOPARTICLE

NANOPARTICULE MÉTALLIQUE FONCTIONNALISÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.01.2016 DE 102016000865**

(43) Veröffentlichungstag der Anmeldung:
**05.12.2018 Patentblatt 2018/49**

(73) Patentinhaber: **FRIZ Biochem Gesellschaft für Bioanalytik mbH**
**82061 Neuried (DE)**

(72) Erfinder: **HARTWICH, Gerhard**
**80639 München (DE)**

(74) Vertreter: **Zeuner Summerer Stütz**
**Patent- und Rechtsanwälte**
**Partnerschaft**
**Nußbaumstraße 8**
**80336 München (DE)**

(56) Entgegenhaltungen:
**WO-A1-2010/081049 WO-A1-2010/120420**
**US-B1- 6 506 564**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 407 872 B1

## Beschreibung

Die Erfindung betrifft allgemein funktionalisierte metallische Nanopartikel und betrifft insbesondere vorfunktionalisierte metallische Nanopartikel als standardisierte Grundbausteine biofunktionalisierter Nanopartikel. Die Erfindung betrifft weiter biofunktionalisierte metallische Nanopartikel sowie einen Nanopartikel-Baukasten zur Herstellung biofunktionalisierter Nanopartikel.

Die biomedizinische Anwendung von Gold-Nanopartikeln nimmt ständig zu, wie weit über 10.000 Publikationen in peer-reviewed Journalen in den letzten 8 Jahren belegen. Gold-Nanopartikel werden beispielsweise als diagnostisches Imaging Agens bei in vivo Untersuchungen oder als therapeutisches Agens beim experimentellen Gen- und Drug Delivery eingesetzt. Insbesondere auf Gold-Nanopartikeln basierende Delivery-Systeme für die Tumor-Chemotherapie wurden ausführlich untersucht, da sie eine höhere Wirkstoff-Effizienz bei geringerer Zytotoxizität für gesundes Gewebe erwartet lassen. Für das Delivery von Nukleinsäuren haben Gold-Nanopartikel gegenüber anderen "synthetischen Vektoren" (Delivery-Systemen) wie Lipid-basierten Vektoren den Vorteil, dass sowohl ihre physikalischen Eigenschaften, beispielsweise ihre Größen- und Oberflächeneigenschaften, als auch ihre chemisch-biologischen Eigenschaften, nämlich insbesondere die multifunktionelle Derivatisierbarkeit der Oberfläche, und damit die Zytotoxizität, Biodistribution und in-vivo-Exkretion nahezu beliebig modulierbar sind.

Diese hohe Modularität funktionalisierter Gold-Nanopartikel bedingt anderseits allerdings auch, dass die Mechanismen für Delivery, Zellgängigkeit, Wirksamkeit und dergleichen in der Literatur noch vielfach kontrovers diskutiert werden, zumal die verschiedenen Forschergruppen jeweils ihre eigenen, sich aufgrund unterschiedlicher Herstellungsverfahren und -parameter unterscheidenden Kompositionen von derivatisierten Gold-Nanopartikeln verwenden.

Gold-Nanopartikel erhält man z.B. durch Reduktion von Tetrachloridogoldsäure $H[AuCl_4]$ in siedender wässriger Lösung mit Citronensäure. Kolloidales nanopartikuläres Gold ist instabil und neigt zur Koagulation / Aggregation. Deswegen werden üblicherweise Stabilisatoren wie zum Beispiel Citrate und/oder Detergentien zugesetzt. Kolloidale Gold-Nanopartikel besitzen im sichtbaren Bereich des Lichts eine starke Oberflächenplasmonen-Absorption, wodurch sie ein großes Potential für photothermische und photometrische Anwendungen haben.

In einer Studie zur Anwendung von Gold-Nanopartikeln im medizinischen Bereich, wie z.B. in Zelluntersuchungen, konnte gezeigt werden, dass die Aufnahme der Nanopartikel durch Endozytose größenabhängig ist. Gold-Nanopartikel mit einer Größe von 45nm werden dabei besser von HeLa-Zellen aufgenommen als 70nm große Gold-Nanopartikel (S.-H. Wang, C.-W. Lee, A. Chiou, P.-K. Wei, Journal of Nanobiotechnology 2010, 8, 33). Es ist daher erforderlich, dass die Nanopartikel in wässrigen, salzhaltigen Lösungen eine stabile Dispersion bilden und keine Agglomerate bilden. In der Regel sind jedoch selbst Citrat- oder Detergens-stabilisierte Gold-Nanopartikel in reinem Zellkulturmedium nicht stabil. Die Folge ist eine starke Agglomeration der Nanopartikel in diesem Medium. Dies kann einerseits durch die Zusätze an Proteinen, wie z.B. BSA (bovine serum albumin) verhindert werden (H. J. Yen, S. H. Hsu, C. L. Tsai, Small 2009, 5, 1553), die den Nanopartikel mit einer monomolekularen Proteinlage aus BSA überziehen und zu einer Zunahme des Partikeldurchmessers um ca. 4-6 nm führen. Andererseits hindert diese Protein-Umhüllung der Goldnanopartikel eine gezielte chemische Derivatisierung der Partikel in wässrigen oder nichtwässrigen Systemen.

Aus der Druckschrift EP 1 301 625 B1 ist ein Verfahren und ein Kit zum Detektieren eines Analyten in einer Probe bekannt, bei dem metallische Nanopartikel-Konjugate verwendet werden, an die Oligonukleotide gebunden sind. Aus der US 2010/0167290 A1 sind molekülmodifizierte Gold-Nanopartikel bekannt, die kovalent gebundene Oligonukleotide umfassen.

Ausgehend davon liegt der Erfindung die Aufgabe zugrunde, die Nachteile des Stands der Technik zu vermeiden, und insbesondere metallische Nanopartikel bereitzustellen, die einfach, schnell und dennoch hochreproduzierbar mit einer gewünschten Biofunktionalisierung versehen werden können. Die Erfindung soll auch solchermaßen biofunktionalisierte metallische Nanopartikel bereitstellen.

Unter einer Biofunktionalisierung wird dabei wie üblich die Anpassung von Eigenschaften eines Stoffs für sichere biomedizinische Anwendungen verstanden.

Diese Aufgabe wird durch die Merkmale der unabhängigen Ansprüche gelöst. Weiterbildungen der Erfindung sind Gegenstand der abhängigen Ansprüche.

Die Erfindung stellt ein vorfunktionalisiertes metallisches Nanopartikel als standardisierten Grundbaustein biofunktionalisierter Nanopartikel bereit. Das vorfunktionalisierte metallische Nanopartikel umfasst ein thiolreaktives metallisches Nanopartikel, das durch ein bifunktionelles Molekül vorfunktionalisiert ist, welches aus einem Ankerbestandteil und einem kurzen Weiterfunktionalisierungsstummel besteht. Dabei ist vorgesehen, dass

- der Ankerbestandteil eine oder mehrere Dithiol-Phosphat-Gruppen umfasst, und

- der kurze Weiterfunktionalisierungsstummel für die Anbindung einer gewünschten Biofunktionalisierung eingerichtet ist und ausgewählt ist aus der Gruppe bestehend aus

i) einem unmodifizierten standardisierten Oligonukleotid-Strang mit 2 bis 18 Basen zur Weiterfunktionalisierung mit Biomolekülen mit einem terminalen komplementären Gegenstrang des standardisierten Oligonukleotid-Strangs, und

ii) einem 2 bis 18 Basen langen Oligonukleotid-Strang, der mit einer terminalen reaktiven Gruppe für Biomoleküle modifiziert ist.

Der Begriff Oligonukleotid ist dabei ein Äquivalent zu dem Begriff Nukleinsäure-Oligomer und bezeichnet eine Nukleinsäure nicht näher spezifizierter Basenlänge. Eine Nukleinsäure umfasst wenigstens zwei kovalent verbundene Nukleotide oder wenigstens zwei kovalent verbundene Pyrimidin-(z.B. Cytosin, Thymin oder Uracil) oder Purin-Basen (z.B. Adenin oder Guanin). Der Begriff Nukleinsäure bezieht sich auf ein beliebiges Rückgrat der kovalent verbundenen Pyrimidin- oder Purin-Basen, wie z. B. auf das Zucker-Phosphat Rückgrat der DNA, cDNA oder RNA, auf ein Peptid-Rückgrat der PNA oder auf analoge Strukturen. Der Begriff Basen bezeichnet die natürlich vorkommenden Nucleobasen Cytosin, Thymin, Uracil, Adenin und Guanin, und auch deren künstliche Analoga solange diese zur Hybridisierung mit komplementären Oligonukleotiden geeignet sind.

Als thiolreaktives metallisches Nanopartikel wird dabei ein metallisches Nanopartikel (M) bezeichnet, das mit Thiolen (HS-R, R= Rest) oder Disulfiden (R-S-S-R) reagiert, beispielsweise unter Ausbildung spontaner, überwiegend chemisorptiver, gegebenenfalls kovalenter oder (teil-)ionischer Wechselwirkungen/Bindung(en) mit den Thiol- (HS-R) oder Disulfid-Gruppen (R-S-S-R') des Bindungspartners zur Adduktbildung M-S-R bzw. M-S-S-R oder R-S-M-...-M-S-R' (wobei die Punkte andeuten, dass nicht notwendigerweise zwei direkt benachbarte M-Atome die Bindung ausbilden). Das thiolreaktive metallische Nanopartikel ohne die Vorfunktionalisierung wird nachfolgend oft auch als Kernbestandteil des Nanopartikels bezeichnet.

Im Fall ii) ist die terminale reaktive Gruppe mit Vorteil ein Alkin-Terminus zur Weiterfunktionalisierung mit Azid-terminierten Biomolekülen, ein Azid-Terminus zur Weiterfunktionalisierung mit Alkin-terminierten Biomolekülen, ist Ni-Nitriloessigsäure zur Weiterfunktionalisierung mit His-Tagterminierten Biomolekülen, oder ist ein Biotin-Terminus zur Weiterfunktionalisierung mit Avidin-terminierten Biomolekülen. Es versteht sich, dass für die terminale reaktive Gruppe und die entsprechende Gegengruppe der Biomoleküle auch andere, dem Fachmann bekannte Paare reaktiver Gruppen in Frage kommen.

Der unmodifizierte standardisierte Oligonukleotid-Strang im Fall i) bzw. der mit einer terminalen reaktiven Gruppe modifizierte Oligonukleotid-Strang im Fall ii) ist dabei mit besonderem Vorteil so ausgewählt, dass das vorfunktionalisierte metallische Nanopartikel lagerstabil ist. Lagerstabilität bedeutet dabei, dass die vorfunktionalisierten metallischen Nanopartikel über einen Zeitraum vom mindestens 4 Wochen, bevorzugt von mindestens 3 Monaten, von mindestens 6 Monaten oder sogar von mindestens 12 Monaten gelagert werden können, ohne aufgrund des Kontakts mit Luftfeuchtigkeit, Licht und anderen Umwelteinflüssen zu degradieren. Die hohe Lagerstabilität ermöglicht es, die Biofunktionalisierung der Nanopartikeln zur Effizienzsteigerung in zwei Teilschritte 'Vorfunktionalisierung' und 'Nutzerspezifische Weiterfunktionalisierung' zu zerlegen, da die Nanopartikel nach der aufwendigen Vorfunktionalisierung beim Hersteller längere Zeit gelagert und dann an Endnutzer vertrieben werden können. Die Endnutzer können dann die vom Hersteller vorfunktionalisierten Nanopartikeln selbst in einfacher Weise nutzerspezifisch weiterfunktionalisieren, und auch beim Endnutzer können die vorfunktionalisierten Nanopartikel und die weiterfunktionalisierten Nanopartikel über längere Zeit gelagert und nach und nach eingesetzt werden.

Bei der nutzerspezifischen Weiterfunktionalisierung ist das vorfunktionalisierte metallische Nanopartikel mit Vorteil durch einfache Inkubation in wässrigem oder nicht-wässrigem Medium weiterfunktionalisierbar.

Ein bedeutender weiterer Vorteil der Zerlegung in die zwei genannten Teilschritte besteht darin, dass der Endnutzer die eigentliche Biofunktionalisierung dem Hersteller nicht offenlegen muss, sondern diese geheim halten und selbst vornehmen kann.

Für den Weiterfunktionalisierungsstummel ist in der Fallgruppe i) in einer bevorzugten Ausgestaltung vorgesehen, dass der Oligonukleotid-Strang eine Schmelztemperatur oberhalb von 40 °C, vorzugsweise zwischen 40 °C und 70 °C aufweist. Weiter ist der Oligonukleotid-Strang in diesem Fall mit besonderem Vorteil nicht codierend, insbesondere nicht humangenomcodierend. Schließlich ist noch in der Fallgruppe i) weiter vorteilhaft vorgesehen, dass mehr als 60%, bevorzugt mehr als 65%, besonders bevorzugt mehr als 70% der Basen des Oligonukleotid-Strangs Guanin und/oder Cytosin sind.

Der Oligonukleotid-Strang weist dann bei gegebener Basenlänge eine besonders hohe Schmelztemperatur auf.

Das genannte Nanopartikel kann mit nur einem bifunktionellen Molekül vorfunktionalisiert sein, für die meisten Anwendungsfälle ist es aber von Vorteil, wenn das Nanopartikel mit mehreren bifunktionellen Molekülen mit demselben Weiterfunktionalisierungsstummel vorfunktionalisiert ist, wobei die Belegungsdichte vorteilhaft sogar so groß wie möglich gewählt wird. Eine Obergrenze ist in der Praxis dabei durch die in der Regel gewünschte Bedingung gegeben, dass an jedem Weiterfunktionalisierungsstummel noch eine Doppelstrangausbildung möglich sein soll.

In einer vorteilhaften Weiterbildung der Erfindung ist das vorfunktionalisierte Nanopartikel für die Anbindung mehrerer unterschiedlicher Biomoleküle eingerichtet und weist dazu zwei, drei oder vier unterschiedliche bifunktionelle

Moleküle mit einem jeweils anderen standardisierten Weiterfunktionalisierungsstummel auf. Mit Vorteil sind die unterschiedlichen Weiterfunktionalisierungsstummel alle durch standardisierte, aber voneinander verschiedene Oligonukleotid-Stränge nach der oben genannten Fallgruppe i) gebildet. Alternativ können die Fallgruppen i) und ii) auch gemischt sein, so dass beispielsweise ein oder zwei Weiterfunktionalisierungsstummel nach Fallgruppe ii) und ein oder zwei Weiterfunktionalisierungsstummel nach Fallgruppe i) vorliegen.

Abseits der bifunktionellen Moleküle ist die metallische Oberfläche des Nanopartikels mit einer Passivierung, insbesondere über Alkanthiole oder Polyethylenglycole versehen.

Der Ankerbestandteil enthält vorzugsweise genau eine, zwei oder drei Dithiol-Phosphat-Gruppen je bifunktionellem Molekül.

Neben den bisher beschriebenen vorfunktionalisierten metallischen Nanopartikeln enthält die Erfindung auch ein biofunktionalisiertes metallisches Nanopartikel, bei dem ein Biomolekül an den Weiterfunktionalisierungsstummel eines vorfunktionalisierten metallischen Nanopartikels der oben beschriebenen Art angebunden ist. Sie enthält auch ein Verfahren zum Herstellen eines biofunktionalisierten metallischen Nanopartikels, bei dem ein Biomolekül an den Weiterfunktionalisierungsstummel eines vorfunktionalisierten metallischen Nanopartikels der oben beschriebenen Art angebunden wird, und betrifft schließlich auch die Verwendung vorfunktionalisierter metallischer Nanopartikel der oben beschriebenen Art zur Anbindung von Biomolekülen.

Dabei kann sowohl bei dem genannten Nanopartikel, dem Verfahren oder der Verwendung vorgesehen sein, dass das Biomolekül durch ein Nukleinsäure-Oligomer, insbesondere ein einzel- oder partiell doppelsträngiges Nukleinsäure-Oligomer oder ein Protein gebildet ist.

Die Erfindung enthält weiter einen Nanopartikel-Baukasten zur Herstellung biofunktionalisierter Nanopartikel, der als standardisierte Grundbausteine vorfunktionalisierte metallische Nanopartikel enthält, und bei dem jeder Grundbaustein durch ein thiolreaktives metallisches Nanopartikel gebildet ist, das durch ein oder mehrere bifunktionelle Moleküle vorfunktionalisiert ist, welche jeweils aus einem Ankerbestandteil und einem kurzen Weiterfunktionalisierungsstummel besteht. Dabei ist vorgesehen, dass

- der Ankerbestandteil für jedes bifunktionelle Molekül eines Grundbausteins eine oder mehrere Dithiol-Phosphat-Gruppen umfasst,

- der kurze Weiterfunktionalisierungsstummel für jedes bifunktionelle Moleküle eines Grundbausteins für die Anbindung einer gewünschten Biofunktionalisierung eingerichtet ist und aus einem unmodifizierten Oligonukleotid-Strang mit 2 bis 18 Basen zur Weiterfunktionalisierung mit Biomolekülen mit einem terminalen komplementären Gegenstrang des Oligonukleotid-Strangs besteht, und wobei

- der unmodifizierten Oligonukleotid-Strang jedes der bifunktionellen Moleküle eines Grundbausteins aus einer Grundmenge vorbestimmter unmodifizierter Oligonukleotid-Stränge ausgewählt ist, die vier oder weniger unmodifizierte Oligonukleotid-Stränge enthält.

In einer vorteilhaften Ausgestaltung ist dabei vorgesehen, dass der Nanopartikel-Baukasten

- erste standardisierte Grundbausteine enthält, deren bifunktionelle Moleküle nur einen unterschiedlichen Weiterfunktionalisierungsstummel enthalten, der durch einen ersten unmodifizierten Oligonukleotid-Strang aus der genannten Grundmenge gebildet ist, und
- zweite standardisierte Grundbausteine enthält, deren bifunktionelle Moleküle genau zwei unterschiedliche Weiterfunktionalisierungsstummel enthalten, die durch den genannten ersten unmodifizierten Oligonukleotid-Strang und einen zweiten unmodifizierten Oligonukleotid-Strang aus der genannten Grundmenge gebildet sind.

Bevorzugt enthält der Nanopartikel-Baukasten weiter dritte standardisierte Grundbausteine, deren bifunktionelle Moleküle genau drei unterschiedliche Weiterfunktionalisierungsstummel enthalten, die durch den genannten ersten und zweiten unmodifizierten Oligonukleotid-Strang und einen dritten unmodifizierten Oligonukleotid-Strang aus der genannten Grundmenge gebildet sind.

In einer zweckmäßigen Ausgestaltung enthält der Nanopartikel-Baukasten weiter vierte standardisierte Grundbausteine, deren bifunktionelle Moleküle genau vier unterschiedliche Weiterfunktionalisierungsstummel enthalten, die durch den genannten ersten, zweiten und dritten unmodifizierten Oligonukleotid-Strang und den vierten unmodifizierten Oligonukleotid-Strang der genannten Grundmenge gebildet sind.

In allen Ausgestaltungen kann der Nanopartikel-Baukasten Grundbausteine mit unterschiedlicher Partikelgröße und/oder unterschiedlicher thermischer und/oder chemischer Stabilität der verankerten Weiterfunktionalisierungsstummel und/oder unterschiedlicher Belegungsdichte mit den bifunktionellen Molekülen und/oder unterschiedlichem Koadsorbat zur Passivierung der freien metallischen Nanopartikel-Oberfläche enthalten. Beispielsweise können die oben

genannten ersten standardisierten Grundbausteine in verschiedenen Partikelgrößen vorliegen, oder die Weiterfunktionalisierungsstummel der ersten standardisierten Grundbausteine können über eine unterschiedliche große Anzahl an Dithiol-Phosphat-Gruppen (insbesondere 1, 2 oder 3) an die Metalloberfläche angebunden sein.

Die die Grundbausteine bildenden vorfunktionalisierten metallischen Nanopartikel sind dabei vorteilhaft lagerstabil, wie oben bereits genauer beschrieben.

Die vorfunktionalisierten metallischen Nanopartikel und die biofunktionalisierten metallische Nanopartikel weisen als Kernbestandteil vorzugsweise jeweils ein Gold-Nanopartikel auf, wobei allerdings auch andere thiolreaktive Metalle, wie etwa Silber, Platin oder Eisen als Material des Kernbestandteils der Nanopartikel in Frage kommen.

Als Nanopartikel werden im Rahmen dieser Beschreibung Partikel mit einer Abmessung unterhalb von 250 nm bezeichnet, wobei sich die Größenangabe auf den Kernbestandteil, also die Nanopartikel ohne die angebundenen Vor- oder Weiterfunktionalisierungen bezieht. Mit Vorteil liegt die Größe der Nanopartikel sogar unterhalb von 100 nm, insbesondere zwischen 5 nm und 50 nm. Gegenwärtig sind im Wesentlichen kugelförmige Nanopartikel bevorzugt, allerdings können neben kugelförmigen Nanopartikeln auch anders geformte Partikel als Kernbestandteil verwendet werden, beispielsweise ellipsoid-, würfel-, stäbchen- oder polyederförmige Partikel. Bei anisotropen Partikelformen bezieht sich die Größenangabe auf die größte Abmessung des Partikels.

Vorteile der Erfindung sowie weitere Ausführungsbeispiele werden nachfolgend anhand der Figuren erläutert, bei deren Darstellung auf eine maßstabs- und proportionsgetreue Wiedergabe verzichtet wurde, um die Anschaulichkeit zu erhöhen.

Es zeigen:

Fig. 1 in (a) schematisch ein vorfunktionalisiertes Gold-Nanopartikel, das als Grundbaustein biofunktionalisierter Nanopartikel dient, in (b) ein zur Anbindung an den Weiterfunktionalisierungsstummel des Gold-Nanopartikels der Fig. 1 eingerichtetes Biomolekül, und in (c) das durch Anbindung des Biomoleküls von (b) entstandene biofunktionalisierte Gold-Nanopartikel,

Fig. 2 die Herauslösung des Fluorophor-markierte Gegenstrangs aus dem Biomolekül des biofunktionalisierten Nanopartikels der Fig. 1(c) durch eine Ziel-RNA,

Fig. 3 schematisch einen Ausschnitt der Oberfläche des GoldNanopartikels der Fig. 1 im Bereich des angebundenen bifunktionellen Moleküls,

Fig. 4 ein vorfunktionalisiertes Gold-Nanopartikel mit mehreren gleichartigen bifunktionellen Molekülen,

Fig. 5 in (a) und (b) jeweils ein vorfunktionalisiertes GoldNanopartikel mit alternativen Weiterfunktionalisierungsstummeln und einem jeweils entsprechend ausgebildeten anzubindenden Biomolekül,

Fig. 6 in (a) und (b) zwei Ausführungsbeispiele für GoldNanopartikel, an die vier verschiedene bifunktionelle Moleküle mit jeweils anderen Weiterfunktionalisierungsstummeln angebunden sind,

Fig. 7 jeweils links Absorptionsspektren verschiedener wässriggelöster Gold-Nanopartikel-Sorten unmittelbar nach Zugabe von 6fach PBS und die gleiche Lösung nach 120 min; rechts sind jeweils die Differenzspektren gezeigt,

Fig. 8 Messergebnisse zur (Maximal-)Belegung in Abhängigkeit von der Ankerfunktion / Zugänglichkeit,

Fig. 9 Messergebnisse zur mengennormierten Gold-NanopartikelThiol Temperaturstabilität, und

Fig. 10 in (a) eine Durchlichtaufnahme und in (b) eine fluoreszenzmikroskopische Aufnahme einer Makrophage, die zur Expression von miRNA 146a angeregt und mit funktionalisierten GoldNanopartikeln inkubiert wurde.

Die Erfindung wird nun am Beispiel von funktionalisierten Gold-Nanopartikeln erläutert, sie ist allerdings nicht auf die zur Illustration gezeigten Gold-Nanopartikel beschränkt, sondern kann auch bei anderen Nanopartikeln mit thiolreaktiven metallischen Oberflächen, wie etwa Silber- oder Platin-Nanopartikeln eingesetzt werden.

Figur 1(a) zeigt hierzu ein vorfunktionalisiertes Gold-Nanopartikel 10, dessen Funktionalisierung 20 zwar selbst keine Biofunktionalisierung darstellt, es jedoch einem Endnutzer erlaubt, das vorpräparierte Nanopartikel einfach und schnell, beispielsweise durch Inkubation in wässrigem oder nicht-wässrigem Medium, mit einer beliebigen gewünschten Biofunktionalisierung 30 (Fig. 1(b)) zu versehen. Die Funktionalisierung 20 des Nanopartikels 10 wird daher im Rahmen dieser Beschreibung als Vorfunktionalisierung bezeichnet, da sie der gewünschten Biofunktionalisierung vorangeht und diese vorbereitet.

Der Kernbestandteil des vorfunktionalisierten Gold-Nanopartikels 10 ist durch ein im Wesentlichen kugelförmiges Gold-Nanopartikel 12 mit einem Durchmesser von etwa 15 nm gebildet. Das kugelförmige Gold-Nanopartikel 12 ist durch ein bifunktionelles Molekül 20 vorfunktionalisiert, welches in seiner ersten Funktion aus einen Ankerbestandteil 22 zur Verankerung an der Gold-Oberfläche des Kernbestandteils und in seiner zweiten Funktion aus einem kurzen Weiterfunktionalisierungsstummel 24 besteht, welcher für die Anbindung der gewünschten Biofunktionalisierung eingerichtet ist.

Der in Fig. 1 nur schematisch dargestellte Ankerbestandteil 22 umfasst dabei eine oder mehrere Dithiol-Phosphat-Gruppen, die eine stabile Verankerung des Moleküls 20 an der Goldoberfläche des Nanopartikels 12 gewährleisten, wie weiter unten in Zusammenhang mit Fig. 3 näher erläutert.

Der mit dem Ankerbestandteil 22 verbundene kurze Weiterfunktionalisierungsstummel 24 ist im Ausführungsbeispiel der Fig. 1 durch einen standardisierten Oligonukleotid-Strang 26 mit vier Basen gebildet. Der Weiterfunktionalisierungsstummel 24 ist selbst nicht codierend, insbesondere nicht humangenomcodierend und dient ausschließlich als spezifische Andockstation für geeignet präparierte Biomoleküle.

In den nicht mit bifunktionellen Molekülen 20 belegten Bereichen ist die freie Gold-Oberfläche des Nanopartikels 12 mit einer Passivierung 14 versehen, im Ausführungsbeispiel etwa über Polyethylenglycole.

Die gewünschten Biomoleküle 30 müssen für die Anbindung an das Nanopartikel 12, wie in Fig. 1(b) gezeigt, einen terminalen komplementären Gegenstrang 36 zu dem Oligonukleotid-Strang 26 des Weiterfunktionalisierungsstummels 24 aufweisen. Der Oligonukleotid-Strang 26 des Gold-Nanopartikels 10 und der terminale komplementäre Gegenstrang 36 des Biomoleküls 30 passen wie Schlüssel und Schloss zueinander, so dass sichergestellt ist, dass nur Biomoleküle 30, die mit einem geeigneten Gegenstrang 36 präpariert worden sind, an das vorfunktionalisierte Gold-Nanopartikel 10 anbinden können.

Im konkreten Ausführungsbeispiel besteht das Biomolekül 30 etwa aus einem längeren Oligonukleotid 32 mit einem gewünschten Nutzbereich 34, an den der bereits genannte terminale komplementäre Gegenstrang 36 anschließt. Im Nutzbereich 34 ist das Oligonukleotid 32 mit einem mit einem Fluorophor 35 markierten Gegenstrang 38 hybridisiert, der zur Genexpressionsanalyse auf eine bestimmte zellspezifischen mRNA zielt.

Eine wesentliche Besonderheit der vorliegenden Erfindung besteht nun darin, dass das Biomolekül 30 der Fig. 1(b) dank der Vorfunktionalisierung durch einfache Pipettierschritte idealerweise in wenigen Minuten an das vorfunktionalisierte Gold-Nanopartikel 10 der Fig. 1(a) angebunden werden kann, wodurch das in Fig. 1(c) gezeigte, mit der gewünschten Biofunktionalisierung versehene Partikel 40 entsteht.

Bei dem im Ausführungsbeispiel gezeigten biofunktionalisierten Nanopartikel 40 ist die Fluoreszenz des angebundenen Fluorophors 35 zunächst durch die unmittelbare Nachbarschaft der Goldoberfläche 12 gelöscht. Trifft das Nanopartikel 40 in einer Zelle auf die Ziel-RNA 42, so wird der Fluorophor-markierte Gegenstrang 38 durch Hybridisierung mit der mRNA aus dem Biomolekül 30 herausgelöst (Pfeil 44) und der Fluorophor 35 kann fluorimetrisch detektiert werden, wie in Fig. 2 schematisch dargestellt.

Grundsätzlich kann ein Gold-Nanopartikel 12 natürlich auch ohne Vorfunktionalisierung 20 direkt mit einem gewünschten Biomolekül 30 (ohne den terminalen Gegenstrang 36) funktionalisiert werden, wie diese im Stand der Technik bereits beschrieben ist. Allerdings gelingt eine solche Biofunktionalisierung von Gold-Nanopartikeln in der Regel nur in einer oft mehrtägigen Prozedur durch gut ausgebildetes Personal und geht mit entsprechend hohen Kosten einher. Dies kann insbesondere dann ein Hindernis darstellen, wenn eine größere Zahl unterschiedlicher Biomoleküle 30 an Gold-Nanopartikel angebunden werden sollen oder wenn die Ergebnisse von Biofunktionalisierungen unterschiedlicher Forschergruppen miteinander verglichen werden sollen. Da meist jede Gruppe eigene Kompositionen von derivatisierten Gold-Nanopartikeln verwendet, weisen selbst mit nominal identischen Biomolekülen funktionalisierte Nanopartikel oft unterschiedliche Eigenschaften auf, so dass Ergebnisse verschiedener Forschergruppen teilweise nur schwer miteinander in Beziehung gesetzt werden können.

Hier schaffen die jetzt vorgeschlagenen vorfunktionalisierten Gold-Nanopartikel 10 wirksam Abhilfe. Durch den Einsatz standardisierter Oligonukleotid-Stränge 26 im Weiterfunktionalisierungsstummel 24 kann ein Nanopartikel-Baukasten mit hochreproduzierbarem Grundmaterial mit maßgeschneiderten Eigenschaften für beliebige Anwendungen oder Untersuchungen bereitgestellt werden. Die aufwendige Biofunktionalisierung von Nanopartikeln wird durch die Erfindung in zwei Teilschritte zerlegt, nämlich einerseits eine standardisierte Vorfunktionalisierung und andererseits eine nutzerspezifische Weiterfunktionalisierung.

Die Vorfunktionalisierung stellt dabei als Erstfunktionalisierung der Gold-Nanopartikel den zeitlich, apparativ und personell aufwendigen Teilschritt dar, der großes Know-How und viel Erfahrung mit der Funktionalisierung von Goldoberflächen erfordert. Diese Vorfunktionalisierung wird nun erfindungsgemäß standardisiert nur für eine relativ kleine Anzahl unterschiedlicher Vorfunktionalisierungen in einem Nanopartikel-Baukasten durchgeführt und erfolgt zentral durch einen Anbieter vorfunktionalisierter Nanopartikel. Der Endnutzer bezieht die vorfunktionalisierten Nanopartikel vom Anbieter und muss selbst nicht über die zur Erstfunktionalisierung notwendigen Kenntnisse und apparativen Mittel verfügen. Die Lagerstabilität der vorfunktionalisierten Gold-Nanopartikel stellt sicher, dass die beiden Schritte 'Vorfunktionalisierung' und Weiterfunktionalisierung' zeitlich und räumlich voneinander entkoppelt werden können.

Ausgehend von den vorfunktionalisierten Nanopartikeln kann die Weiterfunktionalisierung durch den Endnutzer dann sehr einfach und schnell durchgeführt werden und kann auch von wenig geschultem Personal mit wenigen Pipettierschritten erfolgen. Dies hat neben dem geringen Arbeits-, Zeit- und Kostenaufwand insbesondere auch den Vorteil, dass der Endnutzer mit demselben Grundmaterial rasch eine Vielzahl unterschiedlich biofunktionalisierter Nanopartikeln herstellen kann, so dass eine hohe Vergleichbarkeit der Ergebnisse gesichert ist. Da die Weiterfunktionalisierung vom Endnutzer selbst durchgeführt werden kann, muss dieser zudem keine möglicherweise sensiblen Informationen über die anzubindenden Biomoleküle an Dritte geben, was gerade bei Forschungs- und Entwicklungsarbeiten von großer Bedeutung sein kann.

Zur genaueren Erläuterung der Funktionsweise des Ankerbestandteils 22 zeigt Fig. 3 schematisch einen Ausschnitt der Oberfläche des Gold-Nanopartikels 12 im Bereich des angebundenen bifunktionellen Moleküls 20. Für die Verankerung wurde der Weiterfunktionalisierungsstummel 24, hier in Form eines standardisierten Oligonukleotid-Strangs 26, an dessen 3'-Ende mittels 1,2-Dithian-4-O-Dimethoxytrityl-5-[(2-cyanoethyl)-N,N-diisopropyl)]-phosphoramidit (DTPA) mit Dithiol-Modifikationen versehen, die einen stabilen Thiol-Anker auf der Gold-Oberfläche das Nanopartikels 12 ausbilden können. Figur 3 zeigt dabei konkret ein Ausführungsbeispiel mit zwei Dithiol-Phosphat-Gruppen. Ist eine geringere thermische und/oder chemische Stabilität der Verankerung gewünscht, kann die Anbindung statt dessen mit einem Ankerbestandteil mit nur einer Dithiol-Phosphat-Gruppe erfolgen, bei einer gewünschten höheren thermischen und/oder chemischen Stabilität kann die Anbindung mit einem Ankerbestandteil mit drei Dithiol-Phosphat-Gruppe erfolgen.

Auf diese Weise kann die thermische und/oder chemische Stabilität der Verankerung des Weiterfunktionalisierungsstummels 24 nach Wunsch unterschiedlich gewählt werden. Ein Nanopartikel-Baukasten kann daher beispielsweise auch Nanopartikel mit gleichem Weiterfunktionalisierungsstummel, aber unterschiedlich stabiler Verankerung des Weiterfunktionalisierungsstummels enthalten.

Ein vorfunktionalisiertes Gold-Nanopartikel 10 kann selbstverständlich nicht nur ein bifunktionelles Molekül enthalten, wie der einfacheren Darstellung halber in Fig. 1 gezeigt. Vielmehr werden für eine gewünschte Weiterfunktionalisierungsfunktion vorteilhaft mehrere gleichartige bifunktionelle Moleküle 20 an ein Gold-Nanopartikel 12 angebunden, wie in Fig. 4 illustriert. Die Belegungsdichte wird dabei mit Vorteil sogar so groß wie möglich gewählt, was in der Praxis bedeutet, dass die Belegungsdichte so groß gewählt wird, dass an jedem Weiterfunktionalisierungsstummel gerade noch eine Doppelstrangausbildung möglich ist.

Neben den bisher beschriebenen Weiterfunktionalisierungsfunktionen in Form eines Oligonukleotid-Strangs kommen mit Bezug auf Fig. 5(a) auch Gestaltungen in Betracht, bei denen der Weiterfunktionalisierungsstummel 24' eines vorfunktionalisierten Gold-Nanopartikels 12 aus einem ultrakurzen Oligonukleotid 50 mit einem Alkin-Terminus 52 besteht. Die anzubindenden Biomoleküle 30' müssen in diesem Fall mit einem Azid-Terminus 54 versehen werden, wie in Fig. 5(a) ebenfalls schematisch dargestellt. Das der Anbindung des Alkin-Terminus 52 an den Ankerbestandteil 22 dienende ultrakurze Oligonukleotid 50 weist 2 bis 18, vorzugsweise sogar nur 2 bis 8 Basen auf.

Die kupferkatalysierte Alkin-Azid-Cycloaddition (CuAAC) ist ein Beispiel sogenannter Click-Reaktionen. Sie ist stark selektiv und läuft praktisch nur zwischen den Komponenten Azid und Alkin ab. Die Reaktion wird auch durch die meisten anderen organischen Gruppen, die in anzubindenden Biomolekülen vorkommen, wie zum Beispiel Amino- oder Carboxylgruppen, nicht beeinträchtigt. Zudem kommen in nativen Biomolekülen Azide und Alkine nicht vor. Alkin-Modifikationen können beispielsweise in einer Standard-Oligosynthese mit Alkinphosphoramidit erzeugt werden. Mit Azid- und Alkingruppen markierte Proteine können etwa unter Verwendung von aminoreaktivem Azid-NHS-Ester bzw. Alkin-NHS-Ester, oder biotechnologisch mit den entsprechenden modifizierten Aminosäurebausteinen hergestellt werden.

Eine weitere Möglichkeit besteht darin, den Weiterfunktionalisierungsstummel 24" eines vorfunktionalisierten Gold-Nanopartikels 12 aus einem ultrakurzen Oligonukleotid 60 und einer terminalen Ni-Nitriloessigsäure 62 auszubilden. Die anzubindenden Biomoleküle 30" müssen in diesem Fall einen His-Tag-Terminus 64 aufweisen, wie in Fig. 5(b) schematisch gezeigt. Auch bei dieser Variante weist das der Anbindung des Ni-Nitriloessigsäure-Terminus 62 an den Ankerbestandteil 22 dienende ultrakurze Oligonukleotid 60 nur 2 bis 18, vorzugsweise sogar nur 2 bis 8 Basen auf.

Die Ausgestaltungen der Fig. 5 nutzen für die Anbindung der Biomoleküle ebenfalls das Schlüssel-Schloss-Prinzip, da der Alkin-Terminus 52 des Weiterfunktionalisierungsstummels 24' des Gold-Nanopartikels 10 und der Azid-Terminus 54 der Biomoleküle 30' (Fig. 5a), bzw. die terminale Ni-Nitriloessigsäure 62 des Weiterfunktionalisierungsstummels 24" des Gold-Nanopartikels 10 und der His-Tag-Terminus 64 des Biomoleküls 30" (Fig. 5b) jeweils wie Schlüssel und Schloss zueinander passen. Dadurch ist sichergestellt, dass nur jeweils entsprechend modifizierte Biomoleküle 30' bzw. 30" mit ihrem jeweiligen "Schlüssel" 54 bzw. 64 an das am vorfunktionalisierten Gold-Nanopartikel angebrachte "Schloss" 52 bzw. 62 anbinden können.

Die vorfunktionalisierten Gold-Nanopartikel können auch mehrere, unterschiedliche Weiterfunktionalisierungsstummel aufweisen um gezielt mit unterschiedlichen Biomolekülen weiterfunktionalisierbar zu sein. Wie in Fig. 6 illustriert, können an ein Gold-Nanopartikel hierzu bifunktionelle Moleküle angebunden sein, die insbesondere zwei, drei oder vier unterschiedliche Weiterfunktionalisierungsstummel aufweisen. Bei dem Ausführungsbeispiel der Fig. 6(a) enthält das vorfunktionalisierte Gold-Nanopartikel 70 vier unterschiedliche bifunktionelle Moleküle mit einem jeweils anderen Weiterfunktionalisierungsstummel. Die Weiterfunktionalisierungsstummel sind in Fig. 6 exemplarisch alle durch standardisierte, aber voneinander verschiedene Oligonukleotid-Stränge 72, 74, 76, 78 mit jeweils 3 bis 5 Basen gebildet.

Das Ausführungsbeispiel der Fig. 6(b) zeigt ein vorfunktionalisiertes Gold-Nanopartikel 80, bei dem zwei der bifunktionellen Moleküle Weiterfunktionalisierungsstummel in Form unterschiedlicher standardisierter Oligonukleotid-Stränge 82, 84 aufweisen, während ein dritter Weiterfunktionalisierungsstummel aus einem ultrakurzen Oligonukleotid 86 mit einem Alkin-Terminus 88 besteht und ein vierter Weiterfunktionalisierungsstummel aus einem ultrakurzen Oligonukleotid 90 mit einer terminalen Ni-Nitriloessigsäure 92 besteht.

Während bisher zur Erläuterung des erfindungsgemäßen Prinzips jeweils nur einzelne, vorfunktionalisierte Gold-Nanopartikel gezeigt wurden, enthält die Erfindung insbesondere auch einen Nanopartikel-Baukasten zur Herstellung biofunktionalisierter Nanopartikel. Ein solcher Baukasten enthält eine Anzahl von standardisierten Grundbausteinen in Form vorfunktionalisierter Gold-Nanopartikel der oben beschriebenen Art. Um die genannte Standardisierung zu erreichen, wird dabei eine Grundmenge von Oligonukleotid-Strängen vorgegeben, die nur ein, zwei, drei, oder vier Elemente enthält. Beispielsweise wird eine Grundmenge M vorgegeben, die vier unterschiedliche Oligonukleotid-Stränge A, B, C, D mit jeweils 2 bis 18 Basen enthält.

Wie oben bereits allgemein erläutert, sind die Oligonukleotid-Stränge A, B, C, D mit Vorteil nicht codierend, insbesondere nicht humangenomcodierend, und weisen eine Schmelztemperatur oberhalb von 40 °C, insbesondere zwischen 40 °C und 70 °C auf. Dabei können insbesondere mehr als 60%, 65% oder sogar 70% der Basen der Oligonukleotid-Stränge Guanin und/oder Cytosin sein.

Jedes vorfunktionalisierte Gold-Nanopartikel, das als standardisierter Grundbaustein des Baukastens in Frage kommt, ist durch ein oder mehrere bifunktionelle Moleküle der oben beschriebenen Art vorfunktionalisiert, wobei die kurzen Weiterfunktionalisierungsstummel der bifunktionellen Moleküle ausschließlich aus der Grundmenge M ausgewählt sind, also durch einen oder mehrere der Oligonukleotid-Stränge A, B, C oder D gebildet sein müssen.

Konkret enthält ein erfindungsgemäßer Nanopartikel-Baukasten als erste standardisierte Grundbausteine $B_1$ vorfunktionalisierte Gold-Nanopartikel, deren bifunktionelle Moleküle nur einen unterschiedlichen Weiterfunktionalisierungsstummel enthalten, der durch einen Oligonukleotid-Strang aus der genannten Grundmenge M gebildet ist. Es gibt somit vier erste Grundbausteine, nämlich

$$B_1 = \{\text{Au-NP:A, Au-NP:B, Au-NP:C, Au-NP:D}\},$$

wobei die Bezeichnung Au-NP:X bedeutet, dass der oder die Weiterfunktionalisierungsstummel des Gold-Nanopartikels (=Au-NP) durch den Oligonukleotid-Strang X gebildet ist. Es versteht sich, dass ein vorfunktionalisiertes Gold-Nanopartikel Au-NP:X mehrere gleichartige bifunktionelle Moleküle mit demselben Oligonukleotid-Strang X enthalten kann, um die Belegungsdichte zu erhöhen. Der einfachste Nanopartikel-Baukasten $K_1$ besteht nun nur aus den vier Grundbausteinen $B_1$, also $K_1 = B_1$.

Der nächst komplexeren Baukasten erhält man, wenn zu den ersten Grundbausteinen $B_1$ zweite standardisierte Grundbausteine hinzugefügt werden, bei denen die bifunktionellen Moleküle genau zwei unterschiedliche Weiterfunktionalisierungsstummel enthalten, die durch einen ersten bzw. zweiten Oligonukleotid-Strang aus der genannten Grundmenge M gebildet sind. Es gibt somit sechs zweite Grundbausteine, nämlich

$$B_2 = \{\text{Au-NP:A,B, Au-NP:A,C, Au-NP:A,D}$$
$$\text{Au-NP:B,C, Au-NP:B,D, Au-NP:C,D}\},$$

wobei die Bezeichnung Au-NP:X,Y entsprechend bedeutet, dass die Weiterfunktionalisierungsstummel des Gold-Nanopartikels zum Teil durch den Oligonukleotid-Strang X und zum Teil durch den Oligonukleotid-Strang Y gebildet sind. Der komplexere Nanopartikel-Baukasten $K_2$ besteht somit aus den zehn ersten und zweiten Grundbausteinen $K_2 = B_1$ U $B_2$.

Ein dritter Baukasten enthält neben den Grundbausteinen $B_1$ und $B_2$ noch dritte standardisierte Grundbausteine $B_3$, deren bifunktionelle Moleküle genau drei unterschiedliche Weiterfunktionalisierungsstummel enthalten, die durch drei verschiedene Oligonukleotid-Stränge aus der genannten Grundmenge M gebildet sind. Es gibt vier derartige dritte Grundbausteine, nämlich

$$B_3 = \{\text{Au-NP:A,B,C, Au-NP:A,B,D, Au-NP:A,C,D, Au-NP:B,C,D}\},$$

so dass der dritte Baukasten $K_3 = B_1 \cup B_2 \cup B_3$ vierzehn Grundbausteine enthält. Schließlich enthält der vollständige Baukasten $K_4$ auf Grundlage der Grundmenge M zusätzlich zu den genannten Grundbausteinen $B_1$, $B_2$ und $B_3$ noch den standardisierten Grundbaustein, der alle vier Oligonukleotid-Stränge der Grundmenge M als Weiterfunktionalisierungsstummel enthält,

$$B_4 = \{Au\text{-}NP{:}A,B,C,D\},$$

und

$K_4 = B_1 \cup B_2 \cup B_3 \cup B_4$. Mit einem solchen Baukasten $K_4$ können bis zu vier unterschiedliche Biomoleküle in beliebiger Kombination und Reihenfolge an jeweils passende Grundbausteine des Baukastens angebunden werden. Die Grundbausteine sind standardisiert, da sie nur Oligonukleotid-Stränge aus der vorgegebenen Menge M enthalten.

In einer Abwandlung enthält ein Nanopartikel-Baukasten $K_5$ auf Grundlage der genannten Grundmenge M für jede Anzahl an unterschiedlichen Weiterfunktionalisierungsstummel nur jeweils einen Grundbaustein, also beispielsweise

$$K_5 = \{Au\text{-}NP{:}A,\ Au\text{-}NP{:}A,B,\ Au\text{-}NP{:}A,B,C,\ Au\text{-}NP{:}A,B,C,D\}$$

Bei Verwendung dieses Baukastens $K_5$ müssen die ersten anzubindenden Biomoleküle stets mit dem komplementären Gegenstrang zum Oligonukleotid-Strang A terminiert werden, die zweiten anzubindenden Biomoleküle mit dem komplementären Gegenstrang zum Oligonukleotid-Strang B, die dritten anzubindenden Biomoleküle mit dem komplementären Gegenstrang zum Oligonukleotid-Strang C und die vierten anzubindenden Biomoleküle mit dem komplementären Gegenstrang zum Oligonukleotid-Strang D.

Neben den genannten unterschiedlichen Vorfunktionalisierungen kann ein Nanopartikel-Baukasten auch Nanopartikel unterschiedlicher Partikelgröße, unterschiedlicher thermischer und/oder chemischer Stabilität der Vorfunktionalisierungen, unterschiedlicher Belegungsdichte mit den Vorfunktionalisierungsfunktionen und/oder unterschiedlichem Koadsorbat zur Passivierung enthalten.

Die Herstellung von erfindungsgemäßen vorfunktionalisierten Gold-Nanopartikeln (Au-NP), vorteilhafte Eigenschaften derselben und einige Ausführungs- und Anwendungsbeispiele sind nachfolgend näher beschrieben.

**Beispiel 1: Darstellung von Au-NP-Bausteinen mit Oligonukleotid-AnkerFunktionen**

Zur Derivatisierung von Gold-Nanopartikeln sind mehrere Verfahren bekannt, beispielsweise die sogenannte Aussalzmethode, bei der die Anbindung Thiol modifizierter Oligonukleotide an citrat-stabilisierte Gold-NP in wässriger Lösung dadurch bewerkstelligt wird, dass die Salzkonzentration der Lösung über mehrere Tage erhöht wird.

Eine langsame Zunahme der Salzkonzentration lässt sich aber nicht nur wie bisher üblich durch Zugabe kleiner Salzmengen über einen längeren Zeitraum erreichen, sondern auch durch ein kontinuierliches Eindampfen der Lösung (aufkonzentrieren). Dies kann einfach und reproduzierbar in einem Vakuumkonzentrator (Speed-Vac) erreicht werden. Der Zeitaufwand wird dabei auf etwa 2 Stunden reduziert. Die Isolierung der derivatisierten Au-NP vom Lösungsansatz erfolgt über (mehrmalige) Ultrazentrifugation und Re-Suspendierung der pelletierten Au-NP. Andere Aufreinigungsmethoden über Chromatographie, Size Exclusion etc. sind weitaus aufwendiger als eine (mehrfache) Zentrifugation und weniger vollständig.

Es wurde gefunden, dass auf diese Weise auch routinemäßig gemischte Derivatisierungen gelingen, d.h. die gleichzeitige Anbindung verschiedener thiol-modifizierter Oligonukleotide, wenn diese im entsprechenden Molverhältnis angeboten werden, wobei der Molenbruch x des unterrepräsentierten Oligonukleotids im Verhältnis 1,4 x/sqrt(1-x) eingesetzt werden soll, um eine x zu (1-x) Anbindung zweier verschiedener Oligonukleotide zu erreichen.

Die Passivierung der Au-NP muss, unabhängig von der Art der Passivierung, nach Anbindung der Ankeroligos, idealerweise aber vor der Aufreinigung über Zentrifugation, durch Inkubation mit den Passivierungssubstanzen (i.d.R. Alkanthiole oder Polyethylenglycole, PEG) in der Konzentration 10 mM über ca. 15 min erfolgen. Eine Passivierung gleichzeitig mit der Anbindung der Ankeroligos führt nicht zu einem einheitlichem Anbindungs-und Passivierungsverhalten.

Die Belegungsdichte der Au-NP mit Thiol-Oligonukleotid-Ankern kann durch die Wahl der Oligonukleotid-Konzentrationsverhältnisse 'Oligonukleotide zu unmodifizierte Nanopartikel', aber auch durch die Anzahl der Thiolfunktionen bestimmt werden. Diese Methode ist auch für die Anbindung von Doppelsträngen und komplexen Konstrukten gut geeignet. Die Belegungsdichte der Nanopartikel kann dabei über die Anzahl der Thiolfunktionen und damit über den "footprint" der Ankerfunktion gezielt variiert werden kann. Es gelingen gezielte Variationen der Belegung von 20nm

Nanopartikel mit 1-3,10 (+2), 20 (+2), 40 (+3) und 60 (+3) Anbindungsoligos. Für eine optimale Weiterfunktionalisierung mit der Oligonukleotidmodifizierten Au-NP mit weiteren Oligonukleotiden über Gegenstrang-Hybridisierung ist neben der gezielten Belegungsdichte wichtig, die Oligonukleotid-Derivatisierungen an die citrat-stabilisierten Au-NP in Form von Doppelsträngen anzubinden und den (nicht thiol-modifizierten) Gegenstrang nachträglich vom Au-NP zu entfernen. Dies wird zeitgleich mit der Aufreinigung über Zentrifugation /Isolierung der vorfunktionalisierten Au-NP erreicht, indem die Au-NP bei einer Temperatur zentrifugiert werden, die knapp über dem Schmelzpunkt der anmodifizierten doppelsträngigen Oligonukleotide liegt.

Im durch Zentrifugation erhaltenen Isolat aus Oligonukleotid-derivatisierten Au-NP können noch Au-NP-Edukte enthalten sein; deren Anteil lässt sich - bei gewünschter hoher Belegungsdichte- deutlich reduzieren, wenn die Thiol-modifizierten Oligonukleotide des Lösungsansatzes in deutlichem molarem Überschuss eingesetzt werden (z.B. zehn bis fünfzigfacher molarer Überschuss).

**Beispiel 2: Agglomeration**

Unmodifizierte Au-NP sind in salzhaltigen Lösungen instabil und neigen dazu, zu agglomerieren. Dies führt unter anderem dazu, dass unmodifizierte Au-NP nach einer Pelletierung durch (Ultra-) Zentrifugation nur schwer zu re-suspendieren sind (was wiederum zur Abtrennung der unmodifizierter Au-NPs von (leichter re-suspendierbaren, vide infra) modifizierten Au-NPs ausgenutzt werden kann). Das Ausmaß einer solchen Agglomeration kann einfach anhand der Absorptionseigenschaften suspendierter/ gelöster Au-NP verfolgt werden.

Die Absorption wird durch das Gesetz von Lambert und Beer beschrieben ($A = \log (I_0/I) = \varepsilon cd$; A: Absorption, Io:Intensität des eintretenden Lichtstrahls, I: Intensität des austretenden Lichtstrahls, $\varepsilon$:Absorptions-/Extinktionskoeffizient, c:Konzentration und d:Schichtdicke). Da die optischen Eigenschaften im UV-Vis Spektrum durch die Oberflächenplasmonen des Goldes bestimmt werden, die größenabhängig sind, kann die Agglomeration photospektrometrisch verfolgt werden: Zunehmende Agglomeration äußert sich entsprechend durch eine Verschiebung des Absorptionsmaximums zu längeren Wellenlängen, einer Abnahme der Absorption im Maximum und einer Verbreiterung des Spektrums.

Entsprechende Re-Suspendierungsversuche wurden durchgeführt, bei denen sich in einer ersten Tube citrat-stabilisierte unmodifizierte Gold-NP (1) befinden, in einer zweiten Tube Au-NP, die über drei gekoppelte DTPA Funktionen (abgekürzt "(SS)3") mit Amino-C6-TTT-(SS)3 modifiziert wurden und in einer dritten Tube Au-NP, die mit 5' CCT CCT TTA CCG TGA TT-(SS)3 modifiziert wurden, jeweils nach Trocknung der (gleichkonzentrierten) Lösungen über Nacht in der Vakuumzentrifuge und anschließend 0,2% PEG-SH durch einfaches schütteln re-suspendiert. Die beobachtbare Intensität der Rotfärbung der Lösung ist dabei direkt proportional zum Re-Suspendierungsgrad.

Unbehandelte citrat-stabilisierte Au-NP sind bei niedrigem Salzgehalt der Lösung gut löslich. Bei zunehmender Salzkonzentration agglomerieren jedoch auch citrat-stabilisierte Au-NP. Dies kann durch Modifikation der Au-NP mit Oligonukleotiden weitgehend vermieden werden wie in Fig. 7 dargestellt:

Fig. 7 zeigt jeweils links Absorptionsspektren verschiedener wässrig-gelöster Au-NP-Sorten unmittelbar nach Zugabe von 6fach PBS (Bezugszeichen 100, 102,104) und die gleiche Lösung nach 120 min (Bezugszeichen 110,112,114): Oben: citrat-stabilisierte unmodifizierte Au-NP; Mitte Amino-C6-TTT-(SS)3; Unten Au-NP, die mit 5' CCT CCT TTA CCG TGA TT-(SS)3 modifiziert wurden. In der rechten Bildhälfte sind jeweils die Differenzspektren (Bezugszeichen 120,122,124) gezeigt. (PBS: Phosphate Buffered Saline: 37 mM Natriumchlorid, 2,7 mM Kaliumchlorid und 12 mM Gesamt-Phosphat (in Form von $HPO_4^{2-}$ und $H_2PO_4^-$); der pH-Wert der eingestellten Pufferlösung ist 7,4. Die Eigenschaft als Pufferlösung ermöglicht das Arbeiten bei diesem konstanten pH-Wert, durch die verschiedenen Salze besitzt die Lösung den osmotischen Druck des menschlichen Organismus - isotonische Salzlösung).

**Beispiel 3: (Maximal-) Belegung in Abhängigkeit von der Ankerfunktion/ Zugänglichkeit**

1ml citrat-stabilisierte Au-NP (2,7 pmol) werden mit 5nmol Oligonukleotide umgesetzt (vide supra). Die Oligonukleotide tragen am 3' Ende die unterschiedliche Thiol-Ankergruppe und am 5' Ende Fluoreszein (Abkürzungen für Thiol-Modifikationen der Oligonukleotide: "SH": einfache Thiolmodifizierte Oligonukleotide, $HS-(CH_2)_6$-Oligo; "SS": einfache Dithiol-Oligonukleotid R-S-S-Oligo, hier $HO-(CH_2)_6-S-S-(CH_2)_6$-oligo, "(SS)N, N= 1...3, DTPA-modifizierte Oligonukleotide mit einem DTPA-Anker, zwei bzw. 3 direkt kovalent aneinander gekoppelten DTPA-Funktion).

Die Oligosequenz ist immer die gleiche (5' Fluo-CCT CCT TTA CCG TGA TT-Thiol), die Oligos werden als Einzelstrang eingesetzt (ohne Gegenstrang "GS", letzte Zeile, Bezugszeichen 130 in Fig. 8), mit kurzem GS (5' TCA CCG TAA AGG, mittlere Zeile, Bezugszeichen 132 in Fig. 8) hybridisiert oder hybridisiert mit vollständig komplementären Gegenstrang (5' CAA TCA CGG TAA AGG AGG; langer GS, vorderste Zeile, Bezugszeichen 134 in Fig. 8). Nach Inkubation der Au-NP mit dem jeweiligen Oligos für 2h werden nicht besetzte freie Au-Stellen mit PEG-SH "passiviert" (0,2%, 5min) und die Au-NP über Zentrifugation von der Lösung abgetrennt und in $H_2O$ re-suspendiert.

Danach werden die Konzentrationen der Partikel über UV-vis Messung bestimmt (je ca. 0,2 pmol/ml) und 300$\mu$l der Partikelsuspension werden mit 100$\mu$l 0,1M Kaliumcyanid-Lösung versetzt und 1h bei Raumtemperatur inkubiert,

um die Au-NP aufzulösen analog

$$2\,Au + 4\,CN^- + H_2O + 1/2\,O_2 = 2\,[Au(CN)_2]^- + 2\,OH^-$$

Die rote Farbe der Gold-NP verschwindet, die Fluorophor-markierten Oligonukleotide werden frei und deren Fluoreszenz nicht mehr gequencht. Der Gehalt an fluoreszenzmarkierten Oligonukleotide wird mit dem Fluorimeter bestimmt. Figur 8 zeigt die Messergebnisse, wobei entlang der y-Achse die untereinander korrelierte aber unnormierte Fluoreszenzintensität gezeigt ist.

**Beispiel 4: Temperaturstabilität**

Figur 9 illustriert die Temperaturstabilität. Dabei wurden zur Ermittlung der Temperaturstabilität der verschiedenen Thiol- Ankerfunktionen - wie bei Beispiel 3 geschildert - Oligo-modifizierte Au-NP mit langem (unmarkierten) GS verwendet. Die Partikel werden auf die in Fig. 9 angegebenen Temperaturen T aufgeheizt, die Temperatur wird für 5 min. gehalten und anschließend wird die Fluoreszenz der Lösung ermittelt, wobei die (Grund-) Fluoreszenz der Lösung bei 20°C abgezogen wird. Die so erhaltene, über die Temperaturerhöhungen akkumulierte, Fluoreszenzzunahme sollte daher ausschließlich durch thermisch abgelöste 5' Fluo-CCT CCT TTA CCG TGA TTG-Thiol-Moleküle entstehen, bei denen die Fluoreszenz von Fluorescein nicht mehr durch den Au-NP gelöscht wird. Die in Fig. 9 dargestellte Fluoreszenz ist zudem auf die Partikelkonzentration und die relative Gesamtbeladung pro Partikel bezogen und stellt daher eine untereinander korrelierende, ansonsten nicht normierte thermische Stabilität der Au-Thiol-Bindungen dar.

Wie aus Fig. 9 ersichtlich, sind bis 40 °C alle DTPA Oligos (1-3 Einheiten, SS1, SS2, bzw. SS3) etwa identisch und alle stabiler als die Thiol-Oligos (SH und S-S). Ungewöhnlich erscheint die relativ schwache thermische Stabilität des einfachen DTPA-Ankers (SS)1 ab Temperaturen von 60 °C.

**Beispiel 5: Reaktionen in nicht-wässrigem Medien**

Die (Aktivierung und) Kopplung von Liganden kann nicht immer in Wasser/Puffer durchgeführt werden, da unpolare Liganden in wässrigen Medien oft unzureichend löslich bzw. einige Aktivierungsmethoden nicht mit Wasser kompatibel sind.

Es wurden daher modifizierte Au-NP für imunhistologische Färbungen hergestellt: Citrat stabilisierte Au-NP, $H_2N$-TTT-(SS)3-Au-NP und $H_2N$- CCT CCT TTA CCG TGA TT -(SS)3-Au-NP, je ca. 1,5 pmol in 1 mL werden 2x mit Wasser (Entfernung von Salzen) und anschließend 4 x mit Acetonitril gewaschen und zentrifugiert. Dem Pellet werden 500μl Acetonitril und 10μl Divinylsulfon (DVS) zugefügt; die Gold NP sind unter diesen Bedingungen nicht suspendierbar, sie bilden ein Pellet oder (beim Citrat) eine rote Schicht auf der Wandung des Eppendorff-Gefäßes.

Die Proben wurden dann 1h auf 60 °C erhitzt (1,4-Addition einer Doppelbindung des DVS an OH (Citrat) bzw. $NH_2$). Nach dem Abkühlen auf Zimmertemperatur wird 3 x mit Acetonitril gewaschen, dann in Wasser aufnehmen. Es zeigt sich, dass nur die Oligo-Au-NP suspendierbar sind, während die Citrat-Gold-NP einen blauen Niederschlag auf der Wandung bilden.

**Beispiel 6:**

Weiterfunktionalisierung der in Beispiel 5 DVS-aktivierten Gold-NP: Addition von Alkalischer Phosphatase / Avidin Konjugat an die aktivierte Doppelbindung.

Zu je 500μl 0,03mg/ml DVS-aktiviertem bzw. $H_2N$- CCT CCT TTA CCG TGA TT -(SS)3-Au-NP in Wasser werden je 5μl Alkalischer Phosphatase / Avidin Konjugat (Sigma A-7294) gegeben und 24h bei RT inkubiert. Danach wird 4x mit je 500μl Wasser gewaschen.

Die Beladung der oligo-modifizierten Au-NP mit Alkalischer Phosphatase / Avidin kann über die Anbindung von Biotin-4-Fluoreszein an diese modifizierten NP bestimmt werden (Annahmen: keine Fluoreszenzlöschung, alle 4 Biotin Bindungsstellen des Avidins sind zugänglich): Eine erste Tube enthält Alkalischer Phosphatase (AP) / Avidin Konjugat modifizierte $H_2N$-TTT-(SS)3-Au-NP: 3 AP/ Avidin pro Partikel, eine zweite Tube Alkalischer Phosphatase (AP) / Avidin Konjugat modifizierte $H_2N$- CCT CCT TTA CCG TGA TT -(SS)3-Au-NP: 15 AP/ Avidin pro Partikel.

Die Aktivität der AP wird über die Zugabe von alkalischem AP-Puffer und Substrat (NBT, Nitroblau-tetrazoliumchlorid /BCIP, 5-Brom-4-chlor-3-indolylphosphat) ermittelt. 5 min nach Zugabe von NBT/BCIP ist trotz der höheren Anzahl von AP/ Partikel in der zweiten Tube die Farbentwicklung in der ersten Tube intensiver.

Zur Erläuterung der Aktivierung mit DVS: 1,4-Addition; Die Aktivierung von Alkoholen (ROH) erfolgt typischerweise im nichtwässrigen bei erhöhten Temperaturen, die Aktivierung von Aminen ($RNH_2$) kann wässrig oder nichtwässrig

bei RT erfolgen.

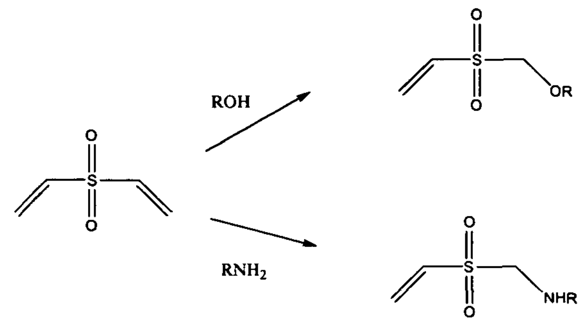


**Beispiel 7: Nachweis von miRNA 146a in Makrophagen mit standardisiertem Au-NP-Baustein**

Makrophagen, die zur Expression von miRNA 146a angeregt wurden, wurden mit 15nm Gold NP inkubiert. Die Gold NP waren wie folgt funktionalisiert:

**5' ACT GAA TTC CAT GGG TT – Cy3 CCT CCT TTA CCG TGA TTG (SS)3 –Au-NP**
**AC TCT TGA CTT AAG GTA CCC AA G GGA GGA AAT GGC ACT AAC**

Diese können einfach mittels des wässrig gut löslichen Au-NP-Bausteins CCT CCT TTA CCG TGA TTG (SS)3 -Au-NP (hergestellt analog Beispiel 1) durch sequentielle oder simultane Inkubation mit den Sequenzen Splint (3'AC TCT TGA CTT AAG GTA CCC AAG GGA GGA AAT GGC ACT AAC) und Target (5' ACT GAA TTC CAT GGG TT - Cy3) erhalten werden. Die Target-Sequenz ACT GAA TTC CAT GGG TT - Cy3 ist komplementär zur gesuchten miRNA 146a der Makrophagen und wird im Addukt bei Vorhandensein der miRNA 146a aus dem Addukt verdrängt. In diesem Fall wird die ansonsten vom Au-NP gelöschte Cy3 Fluoreszenz sichtbar.

Figur 10 zeigt in a) eine Durchlichtaufnahme und in b) eine fluoreszenzmikroskopische Aufnahme einer Makrophage, wobei aus der in Fig. 10b) sichtbaren Fluoreszenz 140 deutlich zu erkennen ist, dass der gewählte Ansatz über den Au-NP-Baustein funktioniert.

Weitere Ausgestaltungen der Erfindung:

Ausgestaltung 1: Vorfunktionalisiertes metallisches Nanopartikel als standardisierter Grundbaustein biofunktionalisierter Nanopartikel, mit einem thiolreaktiven metallischen Nanopartikel, das durch ein bifunktionelles Molekül vorfunktionalisiert ist, welches aus einem Ankerbestandteil und einem kurzen Weiterfunktionalisierungsstummel besteht, wobei

- der Ankerbestandteil eine oder mehrere Dithiol-Phosphat-Gruppen umfasst, und
- der kurze Weiterfunktionalisierungsstummel für die Anbindung einer gewünschten Biofunktionalisierung eingerichtet ist und ausgewählt ist aus der Gruppe bestehend aus

  i) einem standardisierten Oligonukleotid-Strang mit 2 bis 18 Basen zur Weiterfunktionalisierung mit Biomolekülen mit einem terminalen komplementären Gegenstrang des standardisierten Oligonukleotid-Strangs,
  ii) einem 1 bis 4 Basen langen Oligonukleotid mit Alkin-Terminus zur Weiterfunktionalisierung mit Azid-terminierten Biomolekülen, und
  iii) einem 1 bis 4 Basen langen Oligonukleotid mit terminaler Ni-Nitriloessigsäure zur Weiterfunktionalisierung mit His-Tagterminierten Biomolekülen.

Ausgestaltung 2: Vorfunktionalisiertes metallisches Nanopartikel nach Ausgestaltung 1, dadurch gekennzeichnet, dass im Fall i) der Oligonukleotid-Strang eine Schmelztemperatur oberhalb von 40 °C, vorzugsweise zwischen 40 °C und 70 °C aufweist.

Ausgestaltung 3. Vorfunktionalisiertes metallisches Nanopartikel nach Ausgestaltung 1 oder 2, dadurch gekennzeichnet, dass im Fall i) der Oligonukleotid-Strang nicht codierend, insbesondere nicht humangenomcodierend ist.

Ausgestaltung 4. Vorfunktionalisiertes metallisches Nanopartikel nach wenigstens einer der Ausgestaltungen 1 bis

3, **dadurch gekennzeichnet, dass** im Fall i) mehr als 60%, bevorzugt mehr als 65%, besonders bevorzugt mehr als 70% der Basen des Oligonukleotid-Strangs Guanin und/oder Cytosin sind.

Ausgestaltung 5. Vorfunktionalisiertes metallisches Nanopartikel nach wenigstens einer der Ausgestaltungen 1 bis 4, **dadurch gekennzeichnet, dass** das Nanopartikel mit mehreren bifunktionellen Molekülen mit demselben Weiterfunktionalisierungsstummel versehen ist.

Ausgestaltung 6. Vorfunktionalisiertes metallisches Nanopartikel nach wenigstens einer der Ausgestaltungen 1 bis 4, **dadurch gekennzeichnet, dass** das Nanopartikel zwei, drei oder vier unterschiedliche bifunktionelle Moleküle mit einem jeweils anderen standardisierten Weiterfunktionalisierungsstummel aufweist.

Ausgestaltung 7. Vorfunktionalisiertes metallisches Nanopartikel nach wenigstens einer der Ausgestaltungen 1 bis 6, **dadurch gekennzeichnet, dass** die metallische Oberfläche des Nanopartikels abseits der bifunktionellen Moleküle mit einer Passivierung, insbesondere über Alkanthiole oder Polyethylenglycole versehen ist.

Ausgestaltung 8. Biofunktionalisiertes metallisches Nanopartikel, bei dem ein Biomolekül an den Weiterfunktionalisierungsstummel eines vorfunktionalisierten metallischen Nanopartikels nach einer der Ausgestaltungen 1 bis 7 angebunden ist.

Ausgestaltung 9. Verfahren zum Herstellen eines biofunktionalisierten metallischen Nanopartikels, bei dem ein Biomolekül an den Weiterfunktionalisierungsstummel eines vorfunktionalisierten metallischen Nanopartikels nach einer der Ausgestaltungen 1 bis 7 angebunden wird.

Ausgestaltung 10. Verwendung vorfunktionalisierter metallischer Nanopartikel nach einer der Ausgestaltung 1 bis 7 zur Anbindung von Biomolekülen.

Ausgestaltung 11. Nanopartikel, Verfahren oder Verwendung nach Ausgestaltung 8, 9 oder 10, **dadurch gekennzeichnet, dass** das Biomolekül durch ein Nukleinsäure-Oligomer, insbesondere ein einzel- oder partiell doppelsträngiges Nukleinsäure-Oligomer oder ein Protein gebildet ist.

Ausgestaltung 12. Nanopartikel-Baukasten zur Herstellung biofunktionalisierter Nanopartikel, der als standardisierte Grundbausteine vorfunktionalisierte metallische Nanopartikel enthält, und bei dem jeder Grundbaustein durch ein thiolreaktives metallisches Nanopartikel gebildet ist, das durch ein oder mehrere bifunktionelle Moleküle vorfunktionalisiert ist, welche jeweils aus einem Ankerbestandteil und einem kurzen Weiterfunktionalisierungsstummel besteht, wobei

- der Ankerbestandteil für jedes bifunktionelle Molekül eines Grundbausteins eine oder mehrere Dithiol-Phosphat-Gruppen umfasst,
- der kurze Weiterfunktionalisierungsstummel für jedes bifunktionelle Moleküle eines Grundbausteins für die Anbindung einer gewünschten Biofunktionalisierung eingerichtet ist und aus einem Oligonukleotid-Strang mit 2 bis 18 Basen zur Weiterfunktionalisierung mit Biomolekülen mit einem terminalen komplementären Gegenstrang des Oligonukleotid-Strangs besteht, und wobei
- der Oligonukleotid-Strang jedes der bifunktionellen Moleküle eines Grundbausteins aus einer Grundmenge vorbestimmter Oligonukleotid-Stränge ausgewählt ist, die vier oder weniger Oligonukleotid-Stränge enthält.

Ausgestaltung 13. Nanopartikel-Baukasten nach Ausgestaltung 12, dadurch gekennzeichnet, dass der Nanopartikel-Baukasten

- erste standardisierte Grundbausteine enthält, deren bifunktionelle Moleküle nur einen unterschiedlichen Weiterfunktionalisierungsstummel enthalten, der durch einen ersten Oligonukleotid-Strang aus der genannten Grundmenge gebildet ist, und
- zweite standardisierte Grundbausteine enthält, deren bifunktionelle Moleküle genau zwei unterschiedliche Weiterfunktionalisierungsstummel enthalten, die durch den genannten ersten Oligonukleotid-Strang und einen zweiten Oligonukleotid-Strang aus der genannten Grundmenge gebildet sind.

Ausgestaltung 14. Nanopartikel-Baukasten nach Ausgestaltung 13, **dadurch gekennzeichnet, dass** der Nanopartikel-Baukasten weiter

- dritte standardisierte Grundbausteine enthält, deren bifunktionelle Moleküle genau drei unterschiedliche Weiterfunktionalisierungsstummel enthalten, die durch den genannten ersten und zweiten Oligonukleotid-Strang und einen dritten Oligonukleotid-Strang aus der genannten Grundmenge gebildet sind.

Ausgestaltung 15. Nanopartikel-Baukasten nach Ausgestaltung 14, **dadurch gekennzeichnet, dass** Nanopartikel-Baukasten weiter

- vierte standardisierte Grundbausteine enthält, deren bifunktionelle Moleküle genau vier unterschiedliche Weiterfunktionalisierungsstummel enthalten, die durch den genannten ersten, zweiten und dritten Oligonukleotid-Strang und einen vierten Oligonukleotid-Strang aus der genannten Grundmenge gebildet sind.

Ausgestaltung 16. Nanopartikel-Baukasten nach wenigstens einer der Ausgestaltungen 12 bis 15, **dadurch gekennzeichnet, dass** der Nanopartikel-Baukasten Grundbausteine mit unterschiedlicher Partikelgröße und/oder unterschiedlicher thermischer und/oder chemischer Stabilität der verankerten Weiterfunktionalisierungsstummel und/oder unterschiedlicher Belegungsdichte mit den bifunktionellen Molekülen und/oder unterschiedlichem Koadsorbat zur Passivierung enthält.

## Patentansprüche

**1.** Vorfunktionalisiertes metallisches Nanopartikel als standardisierter Grundbaustein biofunktionalisierter Nanopartikel, mit einem thiolreaktiven metallischen Nanopartikel, das durch ein bifunktionelles Molekül vorfunktionalisiert ist, welches aus einem Ankerbestandteil und einem kurzen Weiterfunktionalisierungsstummel besteht, wobei

- der Ankerbestandteil eine oder mehrere Dithiol-Phosphat-Gruppen umfasst, und
- der kurze Weiterfunktionalisierungsstummel für die Anbindung einer gewünschten Biofunktionalisierung eingerichtet ist und ausgewählt ist aus der Gruppe bestehend aus

i) einem unmodifizierten standardisierten Oligonukleotid-Strang mit 2 bis 18 Basen zur Weiterfunktionalisierung mit Biomolekülen mit einem terminalen komplementären Gegenstrang des standardisierten Oligonukleotid-Strangs, und
ii) einem 2 bis 18 Basen langen Oligonukleotid-Strang, der mit einer terminalen reaktiven Gruppe für Biomoleküle modifiziert ist.

**2.** Vorfunktionalisiertes metallisches Nanopartikel nach Anspruch 1, **dadurch gekennzeichnet, dass** im Fall ii) die terminale reaktive Gruppe ein Alkin-Terminus zur Weiterfunktionalisierung mit Azid-terminierten Biomolekülen ist, Ni-Nitriloessigsäure zur Weiterfunktionalisierung mit His-Tagterminierten Biomolekülen, oder ein Biotin-Terminus zur Weiterfunktionalisierung mit Avidin-terminierten Biomolekülen ist.

**3.** Vorfunktionalisiertes metallisches Nanopartikel nach Anspruch 1 oder 2, **dadurch gekennzeichnet, dass** der unmodifizierte standardisierten Oligonukleotid-Strang im Fall i) bzw. der mit einer terminalen reaktiven Gruppe modifizierte Oligonukleotid-Strang im Fall ii) so ausgewählt sind, dass das vorfunktionalisierte metallische Nanopartikel lagerstabil ist.

**4.** Vorfunktionalisiertes metallisches Nanopartikel nach wenigstens einem der Ansprüche 1 bis 3, **dadurch gekennzeichnet, dass** im Fall i) der Oligonukleotid-Strang eine Schmelztemperatur oberhalb von 40 °C, vorzugsweise zwischen 40 °C und 70 °C aufweist.

**5.** Vorfunktionalisiertes metallisches Nanopartikel nach wenigstens einem der Ansprüche 1 bis 4, **dadurch gekennzeichnet, dass** im Fall i) der Oligonukleotid-Strang nicht codierend, insbesondere nicht humangenomcodierend ist.

**6.** Vorfunktionalisiertes metallisches Nanopartikel nach wenigstens einem der Ansprüche 1 bis 5, **dadurch gekennzeichnet, dass** im Fall i) mehr als 60%, bevorzugt mehr als 65%, besonders bevorzugt mehr als 70% der Basen des Oligonukleotid-Strangs Guanin und/oder Cytosin sind.

**7.** Vorfunktionalisiertes metallisches Nanopartikel nach wenigstens einem der Ansprüche 1 bis 6, **dadurch gekennzeichnet, dass** das Nanopartikel mit mehreren bifunktionellen Molekülen mit demselben Weiterfunktionalisierungsstummel versehen ist, oder dass das Nanopartikel zwei, drei oder vier unterschiedliche bifunktionelle Moleküle mit

einem jeweils anderen standardisierten Weiterfunktionalisierungsstummel aufweist.

8. Vorfunktionalisiertes metallisches Nanopartikel nach wenigstens einem der Ansprüche 1 bis 7, **dadurch gekennzeichnet, dass** die metallische Oberfläche des Nanopartikels abseits der bifunktionellen Moleküle mit einer Passivierung, insbesondere über Alkanthiole oder Polyethylenglycole versehen ist.

9. Vorfunktionalisiertes metallisches Nanopartikel nach wenigstens einem der Ansprüche 1 bis 8, **dadurch gekennzeichnet, dass** das vorfunktionalisierte metallische Nanopartikel durch einfache Inkubation in wässrigem oder nichtwässrigem Medium weiterfunktionalisierbar ist.

10. Biofunktionalisiertes metallisches Nanopartikel, bei dem ein Biomolekül an den Weiterfunktionalisierungsstummel eines vorfunktionalisierten metallischen Nanopartikels nach einem der Ansprüche 1 bis 9 angebunden ist.

11. Verfahren zum Herstellen eines biofunktionalisierten metallischen Nanopartikels, bei dem ein Biomolekül an den Weiterfunktionalisierungsstummel eines vorfunktionalisierten metallischen Nanopartikels nach einem der Ansprüche 1 bis 9 angebunden wird.

12. Verwendung vorfunktionalisierter metallischer Nanopartikel nach einem der Ansprüche 1 bis 9 zur Anbindung von Biomolekülen.

13. Nanopartikel, Verfahren oder Verwendung nach Anspruch 10, 11 oder 12, **dadurch gekennzeichnet, dass** das Biomolekül durch ein Nukleinsäure-Oligomer, insbesondere ein einzel- oder partiell doppelsträngiges Nukleinsäure-Oligomer oder ein Protein gebildet ist.

14. Nanopartikel-Baukasten zur Herstellung biofunktionalisierter Nanopartikel, der als standardisierte Grundbausteine vorfunktionalisierte metallische Nanopartikel enthält, und bei dem jeder Grundbaustein durch ein thiolreaktives metallisches Nanopartikel gebildet ist, das durch ein oder mehrere bifunktionelle Moleküle vorfunktionalisiert ist, welche jeweils aus einem Ankerbestandteil und einem kurzen Weiterfunktionalisierungsstummel besteht, wobei

    - der Ankerbestandteil für jedes bifunktionelle Molekül eines Grundbausteins eine oder mehrere Dithiol-Phosphat-Gruppen umfasst,
    - der kurze Weiterfunktionalisierungsstummel für jedes bifunktionelle Moleküle eines Grundbausteins für die Anbindung einer gewünschten Biofunktionalisierung eingerichtet ist und aus einem unmodifizierten Oligonukleotid-Strang mit 2 bis 18 Basen zur Weiterfunktionalisierung mit Biomolekülen mit einem terminalen komplementären Gegenstrang des Oligonukleotid-Strangs besteht, und wobei
    - der unmodifizierte Oligonukleotid-Strang jedes der bifunktionellen Moleküle eines Grundbausteins aus einer Grundmenge vorbestimmter unmodifizierter Oligonukleotid-Stränge ausgewählt ist, die vier oder weniger unmodifizierte Oligonukleotid-Stränge enthält.

15. Nanopartikel-Baukasten nach Anspruch 14, **dadurch gekennzeichnet, dass** der Nanopartikel-Baukasten

    - erste standardisierte Grundbausteine enthält, deren bifunktionelle Moleküle nur einen unterschiedlichen Weiterfunktionalisierungsstummel enthalten, der durch einen ersten unmodifizierten Oligonukleotid-Strang aus der genannten Grundmenge gebildet ist, und
    - zweite standardisierte Grundbausteine enthält, deren bifunktionelle Moleküle genau zwei unterschiedliche Weiterfunktionalisierungsstummel enthalten, die durch den genannten ersten unmodifizierten Oligonukleotid-Strang und einen zweiten unmodifizierten Oligonukleotid-Strang aus der genannten Grundmenge gebildet sind.

16. Nanopartikel-Baukasten nach Anspruch 15, **dadurch gekennzeichnet, dass** der Nanopartikel-Baukasten weiter

    - dritte standardisierte Grundbausteine enthält, deren bifunktionelle Moleküle genau drei unterschiedliche Weiterfunktionalisierungsstummel enthalten, die durch den genannten ersten und zweiten unmodifizierten Oligonukleotid-Strang und einen dritten unmodifizierten Oligonukleotid-Strang aus der genannten Grundmenge gebildet sind, vorzugsweise dass der Nanopartikel-Baukasten weiter
    - vierte standardisierte Grundbausteine enthält, deren bifunktionelle Moleküle genau vier unterschiedliche Weiterfunktionalisierungsstummel enthalten, die durch den genannten ersten, zweiten und dritten unmodifizierten Oligonukleotid-Strang und einen vierten unmodifizierten Oligonukleotid-Strang aus der genannten Grundmenge gebildet sind.

**17.** Nanopartikel-Baukasten nach wenigstens einem der Ansprüche 14 bis 16, **dadurch gekennzeichnet, dass** der Nanopartikel-Baukasten Grundbausteine mit unterschiedlicher Partikelgröße und/oder unterschiedlicher thermischer und/oder chemischer Stabilität der verankerten Weiterfunktionalisierungsstummel und/oder unterschiedlicher Belegungsdichte mit den bifunktionellen Molekülen und/oder unterschiedlichem Koadsorbat zur Passivierung enthält.

**Claims**

**1.** A prefunctionalized metallic nanoparticle as a standardized basic building block of biofunctionalized nanoparticles, having a thiol-reactive metallic nanoparticle which is prefunctionalized by means of a bifunctional molecule which consists of an anchor component and a short further-functionalization-stub, wherein

- the anchor component comprises one or more dithiol phosphate groups, and
- the short further-functionalization-stub is adapted for the attachment of a desired biofunctionalization and is selected from the group consisting of

i) an unmodified standardized oligonucleotide strand containing 2 to 18 bases for further functionalization with biomolecules having a terminal complementary counter-strand of the standardized oligonucleotide strand, and
ii) a 2 to 18 base long oligonucleotide strand which is modified with a terminal reactive group for biomolecules.

**2.** The prefunctionalized metallic nanoparticle according to claim 1, **characterized in that** in case ii), the terminal reactive group is an alkyne terminus for further functionalization with azide terminated biomolecules, Ni-nitriloacetic acid for further functionalization with His-Tag terminated biomolecules, or a biotin terminus for further functionalization with avidin terminated biomolecules.

**3.** The prefunctionalized metallic nanoparticle according to claim 1 or claim 2, **characterized in that** the unmodified standardized oligonucleotide strand in case i) or the oligonucleotide strand modified with a terminal reactive group in case ii) are selected in a manner such that the prefunctionalized metallic nanoparticle is stable upon storage.

**4.** The prefunctionalized metallic nanoparticle according to at least one of claims 1 to 3, **characterized in that** in case i), the oligonucleotide strand has a melting temperature of more than 40°C, preferably between 40°C and 70°C.

**5.** The prefunctionalized metallic nanoparticle according to at least one of claims 1 to 4, **characterized in that** in case i), the oligonucleotide strand is non-coding, and in particular does not code for the human genome.

**6.** The prefunctionalized metallic nanoparticle according to at least one of claims 1 to 5, **characterized in that** in case i), more than 60 %, preferably more than 65%, particularly preferably more than 70 % of the bases of the oligonucleotide strand are guanine and/or cytosine.

**7.** The prefunctionalized metallic nanoparticle according to at least one of claims 1 to 6, **characterized in that** the nanoparticle is provided with a plurality of bifunctional molecules having the same further-functionalization-stub, or **in that** the nanoparticle comprises two, three or four different bifunctional molecules, each having a different standardized further-functionalization-stub.

**8.** The prefunctionalized metallic nanoparticle according to at least one of claims 1 to 7, **characterized in that**, apart from the bifunctional molecules, the metallic surface of the nanoparticle is provided with a passivation, especially via alkane thiols or polyethylene glycols.

**9.** The prefunctionalized metallic nanoparticle according to at least one of claims 1 to 8, **characterized in that** the prefunctionalized metallic nanoparticle is further-functionalizable by means of simple incubation in an aqueous or non-aqueous medium.

**10.** A biofunctionalized metallic nanoparticle, in which a biomolecule is attached to the further-functionalization-stub of a prefunctionalized metallic nanoparticle according to one of claims 1 to 9.

**11.** A method for the production of a biofunctionalized metallic nanoparticle, in which a biomolecule is attached to the

further-functionalization-stub of a prefunctionalized metallic nanoparticle according to one of claims 1 to 9.

**12.** Use of prefunctionalized metallic nanoparticles according to one of claims 1 to 9, for the attachment of biomolecules.

**13.** The nanoparticle, method or use according to claim 10, 11 or 12, **characterized in that** the biomolecule is formed by a nucleic acid oligomer, in particular a single-stranded or partially double-stranded nucleic acid oligomer or a protein.

**14.** A nanoparticle kit for the production of biofunctionalized nanoparticles which contain prefunctionalized metallic nanoparticles as standardized basic building blocks, and in which each basic building block is formed by a thiol-reactive metallic nanoparticle which is prefunctionalized by one or more bifunctional molecules, each of which consists of an anchor component and a short further-functionalization-stub, wherein

- the anchor component for each bifunctional molecule of a basic building block comprises one or more dithiol phosphate groups,
- the short further-functionalization-stub for each bifunctional molecule of a basic building block is adapted for the attachment of a desired biofunctionalization and consists of an unmodified oligonucleotide strand containing 2 to 18 bases for further functionalization with biomolecules having a terminal complementary counter-strand of the oligonucleotide strand, and wherein
- the unmodified oligonucleotide strand of each of the bifunctional molecules of a basic building block is selected from a core set of predetermined unmodified oligonucleotide strands which contains four or fewer unmodified oligonucleotide strands.

**15.** The nanoparticle kit according to claim 14, **characterized in that** the nanoparticle kit contains

- first standardized basic building blocks, the bifunctional molecules therein containing only one different further-functionalization-stub which is formed from said core set by means of a first unmodified oligonucleotide strand, and
- second standardized basic building blocks, the bifunctional molecules therein containing exactly two different further-functionalization-stubs which are formed from said core set by means of said first unmodified oligonucleotide strand and a second unmodified oligonucleotide strand.

**16.** The nanoparticle kit according to claim 15, **characterized in that** the nanoparticle kit further contains

- third standardized basic building blocks, the bifunctional molecules therein containing exactly three different further-functionalization-stubs which are formed from said core set by means of said first and second unmodified oligonucleotide strands and a third unmodified oligonucleotide strand, preferably **in that** the nanoparticle kit further contains
- fourth standardized basic building blocks, the bifunctional molecules therein containing exactly four different further-functionalization-stubs which are formed from said core set by means of said first, second and third unmodified oligonucleotide strands and a fourth unmodified oligonucleotide strand.

**17.** The nanoparticle kit according to at least one of claims 14 to 16, **characterized in that** the nanoparticle kit contains basic building blocks having a different particle size and/or a different thermal and/or chemical stability of the anchored further-functionalization-stubs and/or a different density of coverage with the bifunctional molecules and/or a different co-adsorbate for passivation.

## Revendications

**1.** Nanoparticule métallique préfonctionnalisée, en tant que constituant de base standardisé de nanoparticules bifonctionnalisées, avec une nanoparticule métallique réactive au thiol, qui est préfonctionnalisée par une molécule bifonctionnelle, laquelle est constituée d'un composant d'ancrage et d'un court tronçon de post-fonctionnalisation,

- le composant d'ancrage comprenant un ou plusieurs groupements dithiol phosphate, et
- le court tronçon de post-fonctionnalisation étant aménagé pour la liaison d'une bifonctionnalisation souhaitée et étant choisi dans le groupe comprenant

i) un brin oligonucléotide standardisé non modifié, avec de 2 à 18 bases pour la post-fonctionnalisation avec des biomolécules avec un brin antagoniste terminal complémentaire du brin oligonucléotide standardisé et

ii) un brin oligonucléotide long de 2 à 18 bases qui est modifié avec un groupe terminal réactif pour des biomolécules.

**2.** Nanoparticule métallique préfonctionnalisée selon la revendication 1, **caractérisée en ce que** dans le cas ii), le groupe terminal réactif est un alcyne terminal destiné à la post-fonctionnalisation avec des biomolécules à terminaison azoture, un acide ninitriloacétique pour la post-fonctionnalisation avec des biomolécules à terminaison His-Tag, ou une terminaison biotine pour la post-fonctionnalisation avec des biomolécules à terminaison avidine.

**3.** Nanoparticule métallique préfonctionnalisée selon la revendication 1 ou 2, **caractérisée en ce que** dans le cas i), le brin oligonucléotide standardisé non modifié ou, dans le cas ii), le brin oligonucléotide modifié avec un groupe terminal réactif sont sélectionnés de telle sorte que la nanoparticule métallique préfonctionnalisée soit stable au stockage.

**4.** Nanoparticule métallique préfonctionnalisée selon au moins l'une quelconque des revendications 1 à 3, **caractérisée en ce que** dans le cas i), le brin oligonucléotide fait preuve d'une température de fusion supérieure à 40 °C, comprise de préférence entre 40 °C et 70 °C.

**5.** Nanoparticule métallique préfonctionnalisée selon au moins l'une quelconque des revendications 1 à 4, **caractérisée en ce que** dans le cas i), le brin oligonucléotide est non codant, notamment non codant dans le génome humain.

**6.** Nanoparticule métallique préfonctionnalisée selon au moins l'une quelconque des revendications 1 à 5, **caractérisée en ce que** dans le cas i), plus de 60 %, de préférence plus de 65 %, de manière particulièrement préférentielle, plus de 70 % des bases du brin oligonucléotide sont de la guanine et/ ou de la cytosine.

**7.** Nanoparticule métallique préfonctionnalisée selon au moins l'une quelconque des revendications 1 à 6, **caractérisée en ce que** la nanoparticule est munie de plusieurs molécules bifonctionnelles pourvues du même tronçon de post-fonctionnalisation, ou **en ce que** la nanoparticule comporte deux, trois ou quatre différentes molécules bifonctionnelles pourvues d'un tronçon de post-fonctionnalisation standardisé respectivement autre.

**8.** Nanoparticule métallique préfonctionnalisée selon au moins l'une quelconque des revendications 1 à 7, **caractérisée en ce qu'**en marge des molécules bifonctionnelles, la surface métallique de la nanoparticule est munie d'une passivation, notamment par alcanethiol ou par polyéthylène-glycols.

**9.** Nanoparticule métallique préfonctionnalisée selon au moins l'une quelconque des revendications 1 à 8, **caractérisée en ce que** la nanoparticule métallique préfonctionnalisée est post-fonctionnalisable par simple incubation dans un milieux aqueux ou non aqueux.

**10.** Nanoparticule métallique biofonctionnalisée, pour laquelle une biomolécule est liée à un tronçon de post-fonctionnalisation de nanoparticules métalliques préfonctionnalisées selon l'une quelconque des revendications 1 à 9.

**11.** Procédé, destiné à la fabrication d'une nanoparticule métallique biofonctionnalisée, lors duquel on lie une biomolécule au tronçon de post-fonctionnalisation d'une nanoparticule métallique préfonctionnalisée selon l'une quelconque des revendications 1 à 9.

**12.** Utilisation de nanoparticules métalliques préfonctionnalisées selon l'une quelconque des revendications 1 à 9 pour la liaison de biomolécules.

**13.** Nanoparticule, procédé ou utilisation selon la revendication 10, 11 ou 12, caractérisé(e) en ce que la biomolécule est formée d'un oligomère d'acide nucléique, notamment d'un oligomère d'acide nucléique individuellement ou partiellement à double-brin ou d'une protéine.

**14.** Système modulaire de nanoparticules, destiné à fabriquer des nanoparticules biofonctionnalisées, qui en tant que constituants de base standardisés contient des nanoparticules métalliques préfonctionnalisées, et dans lequel chaque constituant de base est formé d'une nanoparticule métallique réactive au thiol, qui est préfonctionnalisée par une ou plusieurs molécules bifonctionnelles, laquelle est composée respectivement d'un composant d'ancrage et

d'un court tronçon de post-fonctionnalisation,

- le composant d'ancrage pour chaque molécule bifonctionnelle d'un constituant de base comprenant un ou plusieurs groupements dithiol phosphate,
- pour chaque molécule bifonctionnelle d'un constituant de base, le court tronçon de post-fonctionnalisation étant aménagé pour la liaison d'une biofonctionnalisation souhaitée et étant constitué d'un brin oligonucléotide non modifié, avec de 2 à 18 bases pour la post-fonctionnalisation avec des biomolécules avec un brin antagoniste terminal complémentaire du brin oligonucléotide, et
- le brin oligonucléotide non modifié de chacune des molécules bifonctionnelles d'un constituant de base étant choisi parmi une quantité de base de brins oligonucléotide non modifiés prédéterminée qui contient quatre brins oligonucléotide non modifiés ou moins.

**15.** Système modulaire de nanoparticules selon la revendication 14, **caractérisé en ce que** le système modulaire de nanoparticules

- contient des premiers constituants de base standardisés dont les molécules bifonctionnelles ne contiennent qu'un tronçon de post-fonctionnalisation différent, qui est formé d'un premier brin oligonucléotide non modifié de la quantité de base citée, et
- contient des deuxièmes constituants de base standardisés, dont les molécules bifonctionnelles contiennent précisément deux tronçons de post-fonctionnalisation différents, qui sont formés du premier brin oligonucléotide non modifié et d'un deuxième brin oligonucléotide non modifié de la quantité de base citée.

**16.** Système modulaire de nanoparticules selon la revendication 15, **caractérisé en ce que** par ailleurs, le système modulaire de nanoparticules

- contient des troisièmes constituants de base standardisés dont les molécules bifonctionnelles contiennent précisément trois tronçons de post-fonctionnalisation différents, qui sont formés des premier et deuxième brins oligonucléotide non modifiés cités et d'un troisième brin oligonucléotide non modifié de la quantité de base citée, de préférence, **en ce que** par ailleurs, le système modulaire de nanoparticules
- contient des quatrièmes constituants de base standardisés dont les molécules bifonctionnelles contiennent précisément quatre tronçons de post-fonctionnalisation différents, qui sont formés des premier, deuxième et troisième brins oligonucléotide non modifiés cités et d'un quatrième brin oligonucléotide non modifié de la quantité de base citée.

**17.** Système modulaire de nanoparticules selon au moins l'une quelconque des revendications 14 à 16, **caractérisé en ce que** le système modulaire de nanoparticules contient des constituants de base de différentes tailles de particules et/ ou de différente stabilité thermique et/ ou chimique des tronçons de post-fonctionnalisation ancrés et/ ou différentes densités d'occupation par les molécules bifonctionnelles et/ ou un différent coadsorbat pour la passivation.

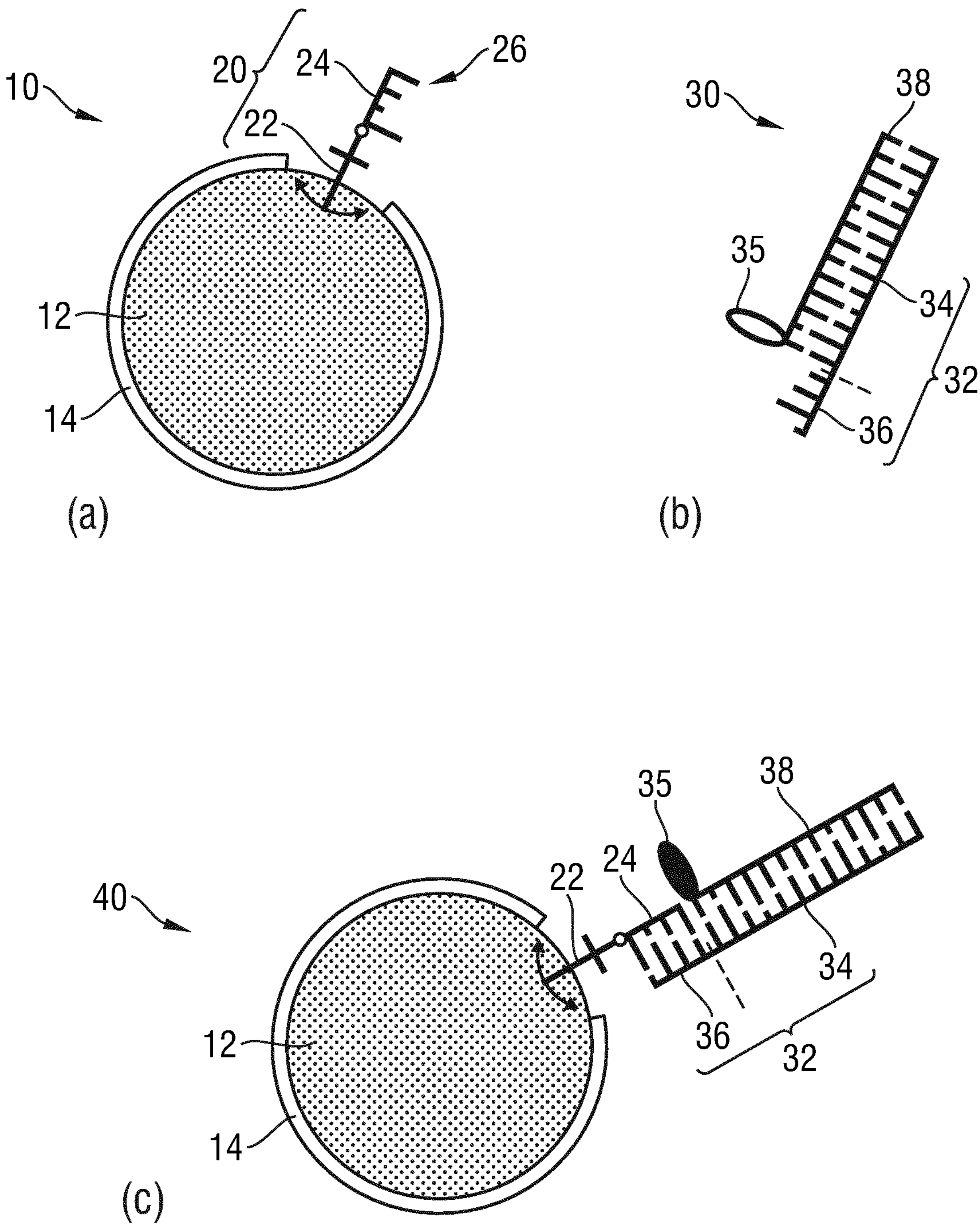


Fig. 1

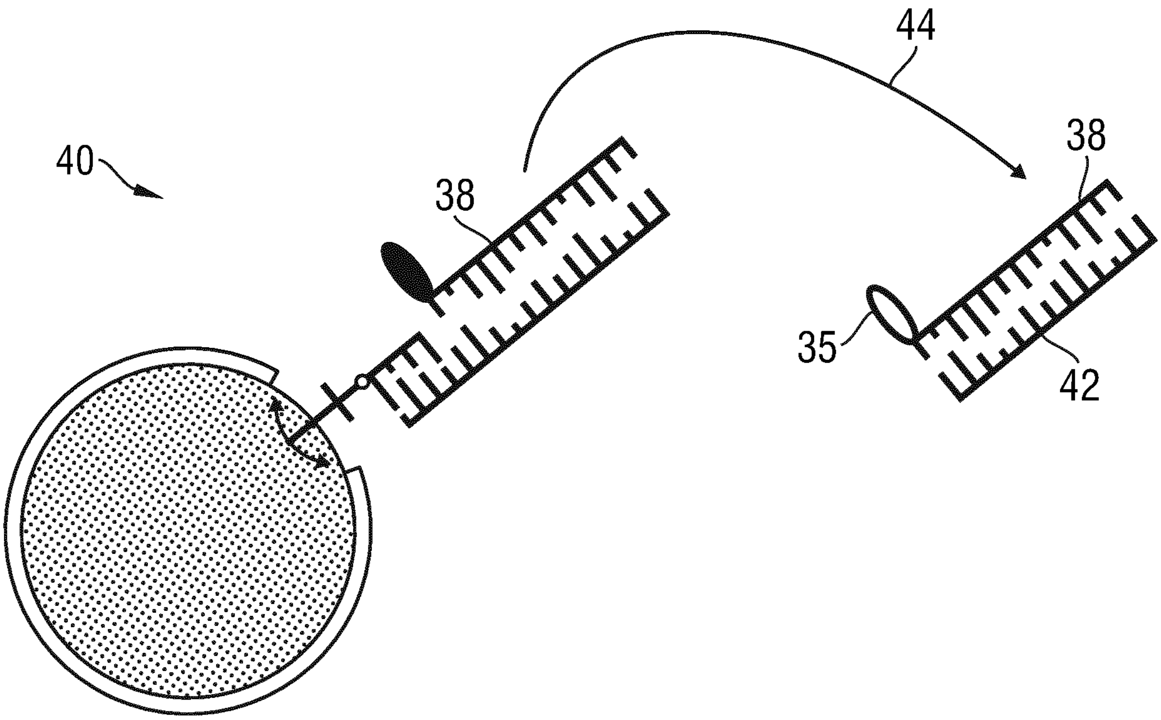


Fig. 2

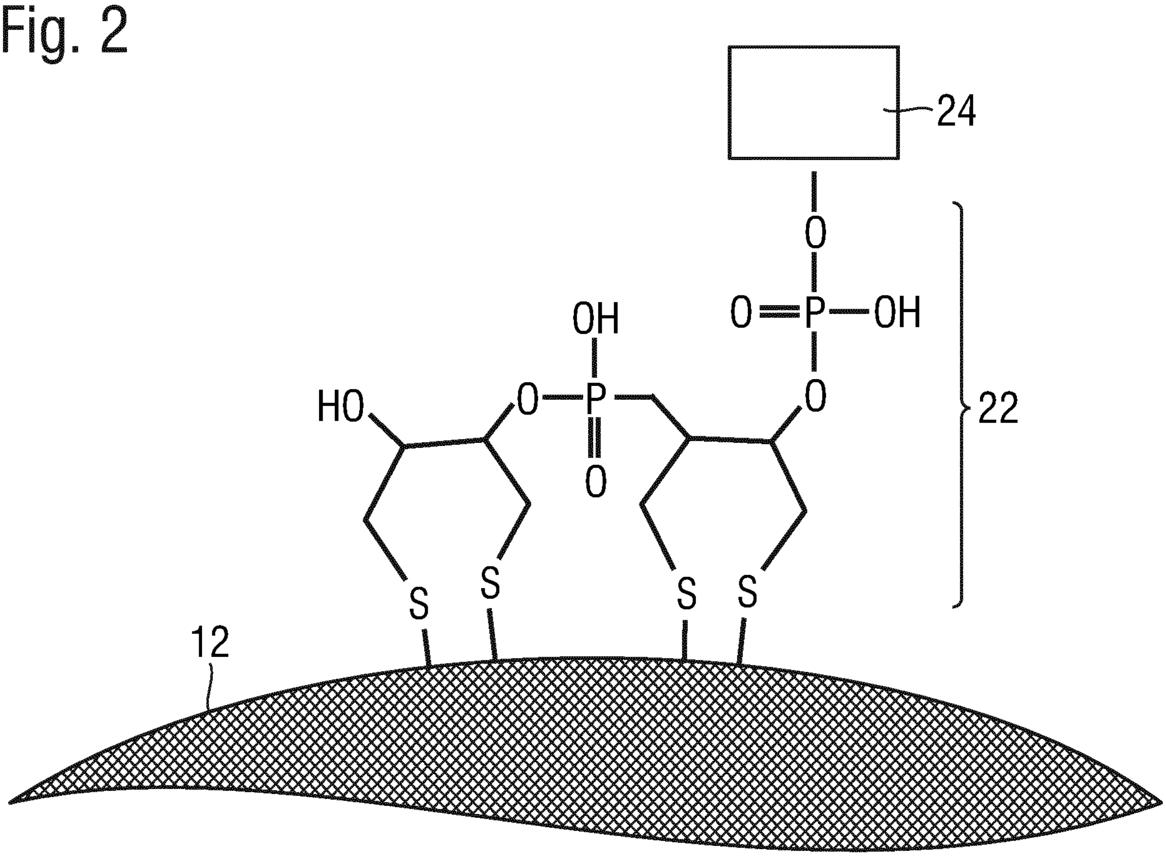


Fig. 3

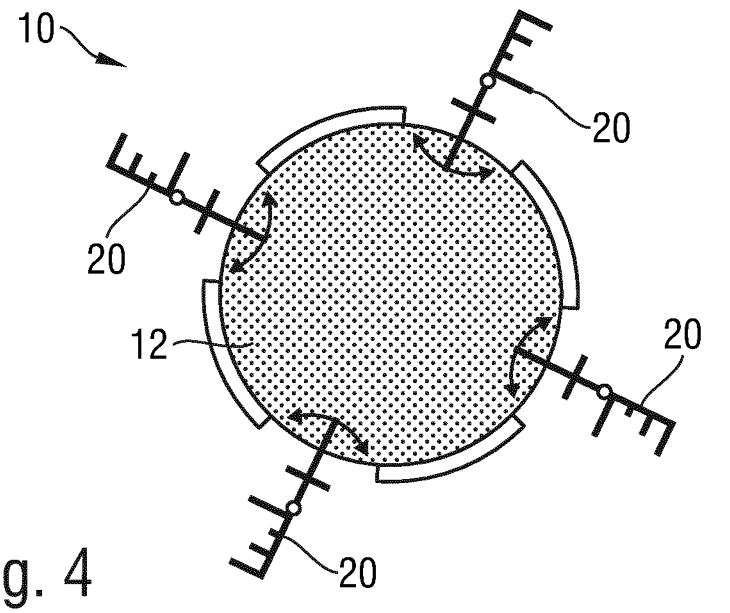


Fig. 4

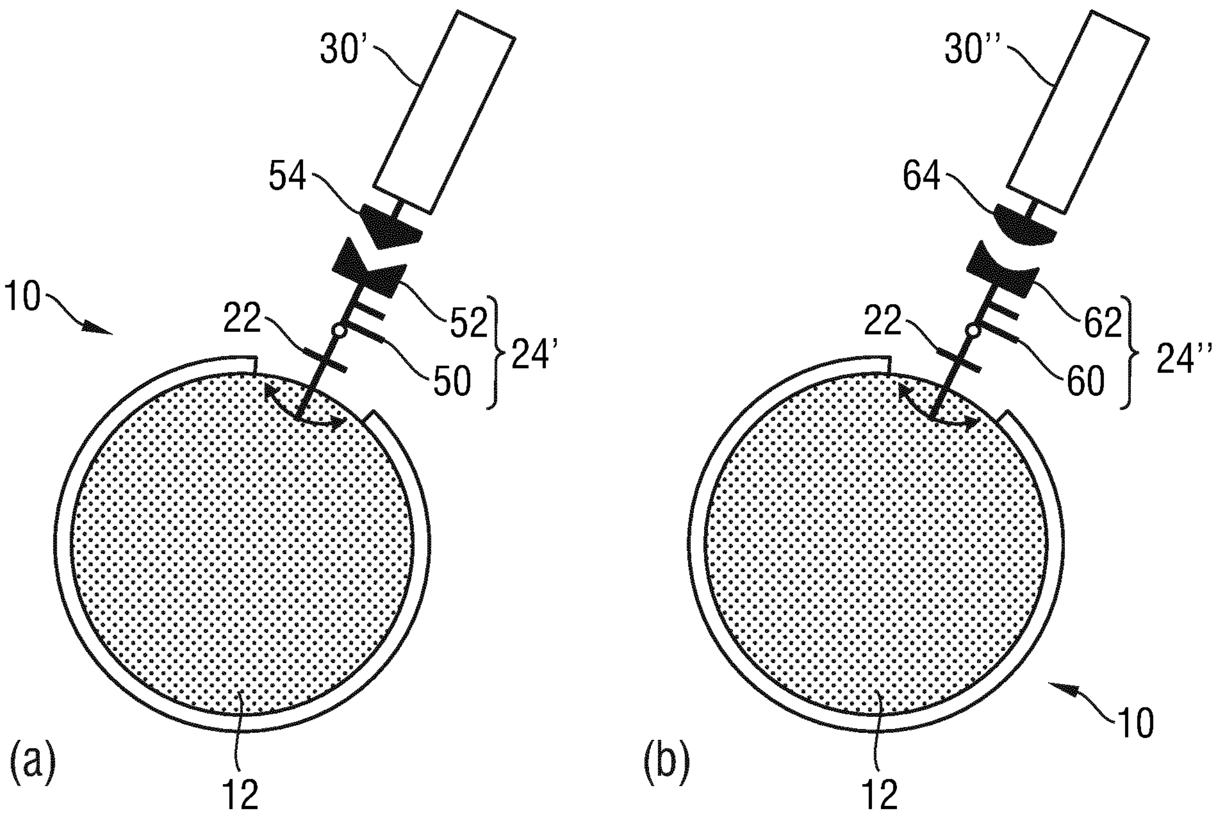


(a)

(b)

Fig. 5

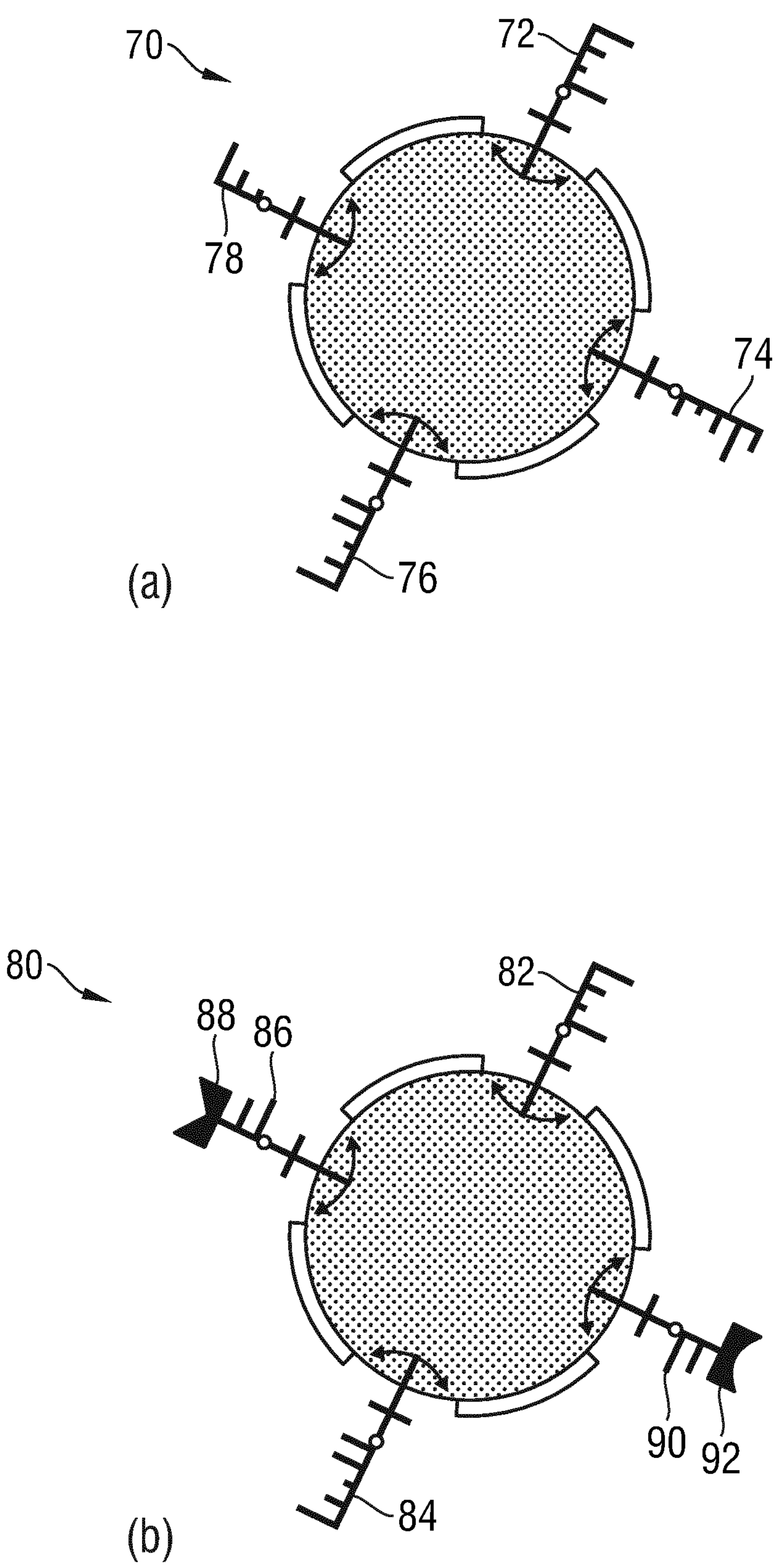


Fig. 6

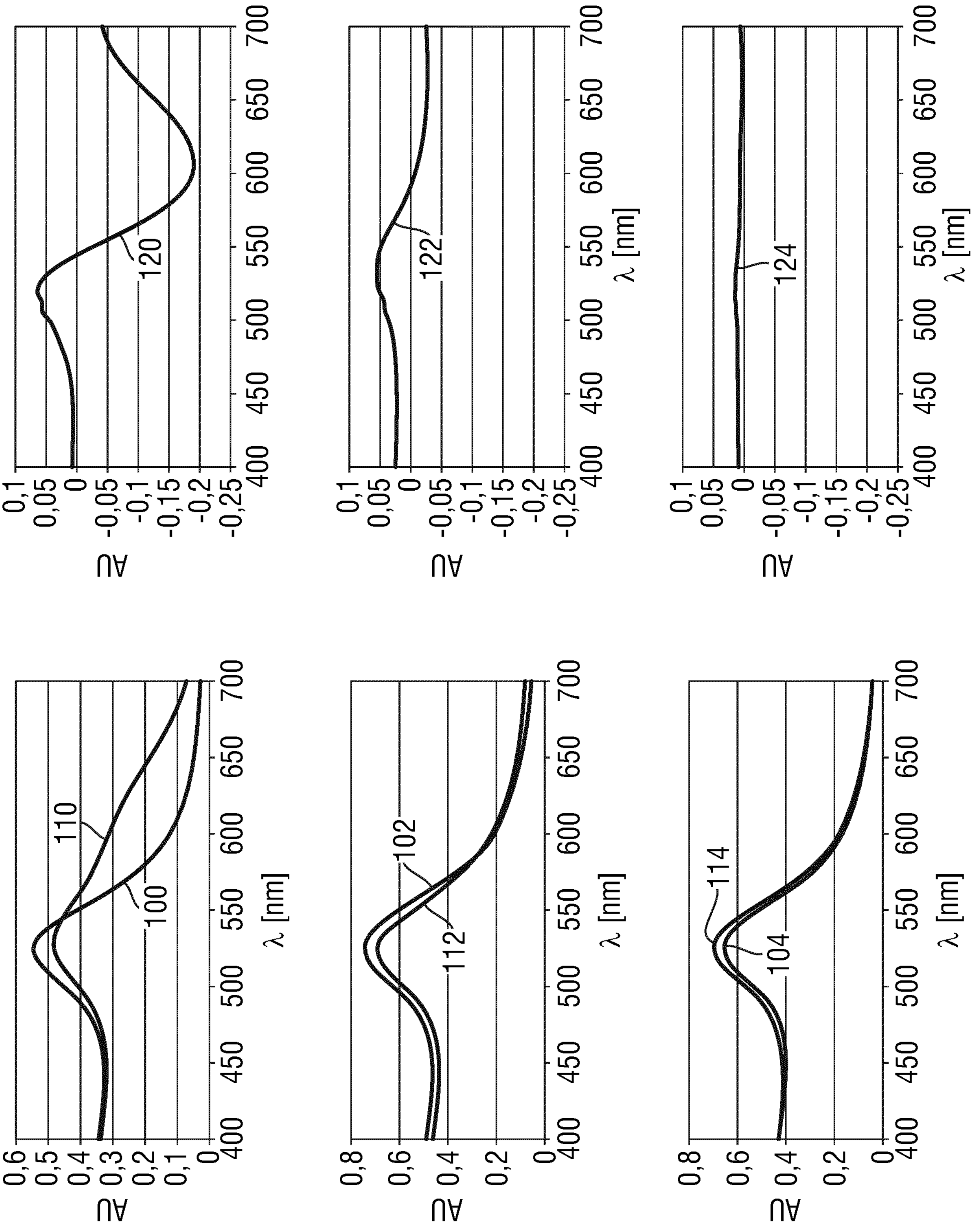
AU
0,6
0,5
0,4
0,3
0,2
0,1
0
400
450
500
550
600
650
700
λ [nm]
100
110
0,8
102
112
104
114
0,1
0,05
-0,05
-0,1
-0,15
-0,2
-0,25
120
122
124

Fig. 7

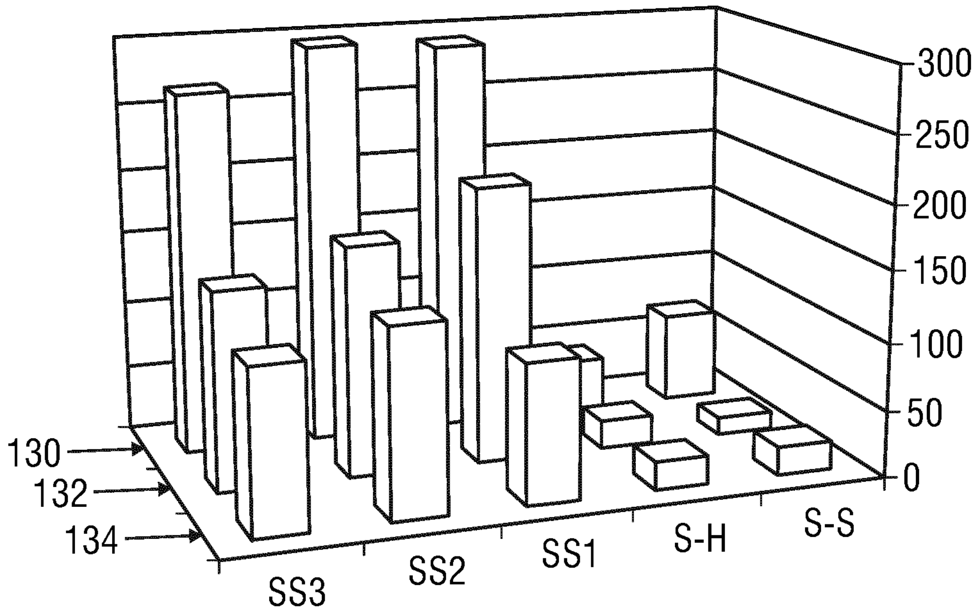
300
250
200
150
100
50
0
130
132
134
SS3
SS2
SS1
S-H
S-S

Fig. 8

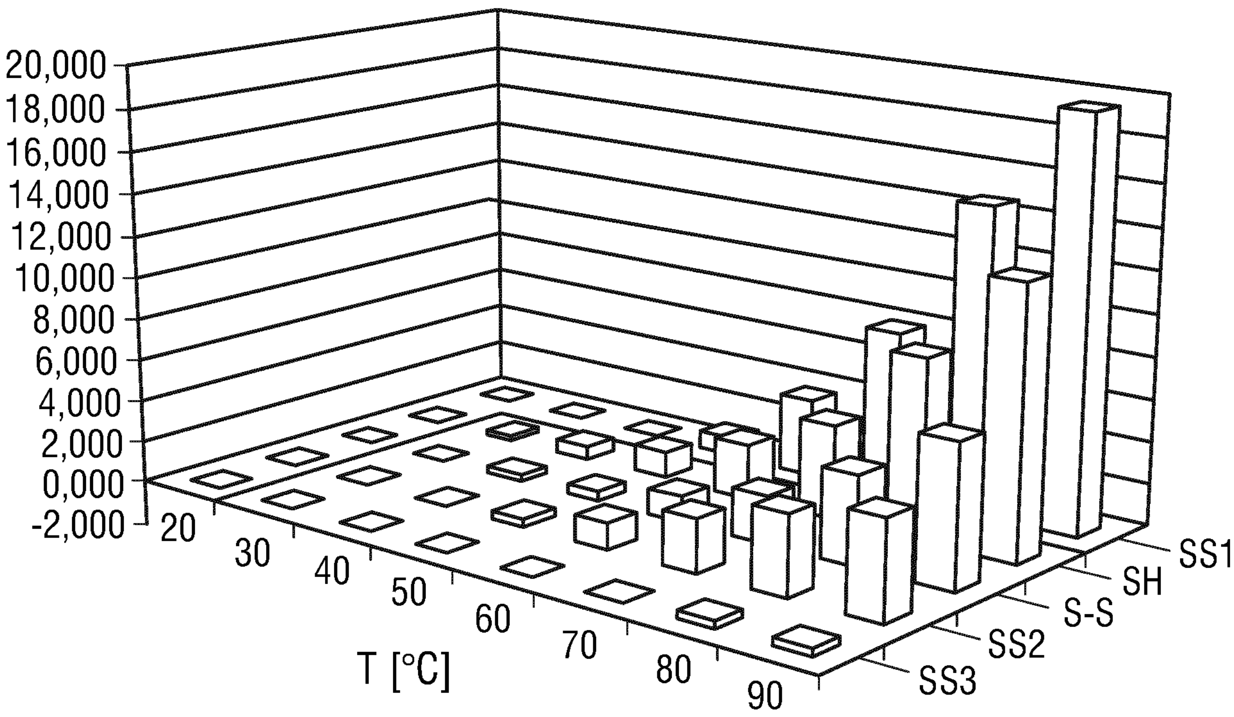
20,000
18,000
16,000
14,000
12,000
10,000
8,000
6,000
4,000
2,000
0,000
-2,000
20
30
40
50
60
70
80
90
T [°C]
SS1
SH
S-S
SS2
SS3

Fig. 9

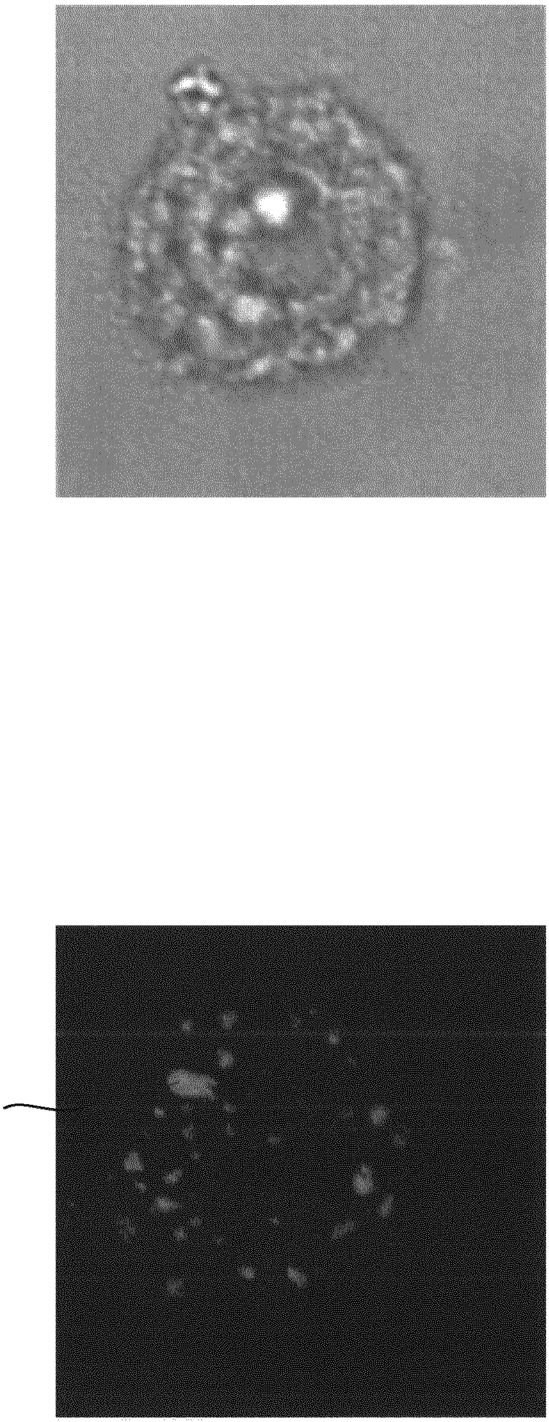

(a)

140

(b)

Fig. 10

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1301625 B1
- US 20100167290 A1


### In der Beschreibung aufgeführte Nicht-Patentliteratur


- **S.-H. WANG ; C.-W. LEE ; A. CHIOU ; P.-K. WEI.** *Journal of Nanobiotechnology,* 2010, vol. 8, 33
- **H. J. YEN ; S. H. HSU ; C. L. TSAI.** *Small,* 2009, vol. 5, 1553